# EUROPEAN PATENT SPECIFICATION

(11) **EP 1 885 725 B1**
(45) Date of publication and mention of the grant of the patent: **24.09.2008**
(21) Application number: 06707595.2
(22) Date of filing: 17.03.2006
(51) Int. Cl.: C07D 487/04, A61K 31/4188, A61P 35/00, A61P 43/00, C07D 209/00, C07D 235/00

(54) **TRICYCLIC AZOLE DERIVATIVES, THEIR MANUFACTURE AND USE AS PHARMACEUTICAL AGENTS**
TRICYCLISCHE AZOLDERIVATE, IHRE HERSTELLUNG UND IHRE VERWENDUNG ALS PHARMAZEUTISCHE MITTEL
DERIVES D'AZOLE TRICYCLIQUES, LEUR FABRICATION ET LEUR UTILISATION EN TANT QUE PRINCIPES PHARMACEUTIQUES

(30) Priority: 14.04.2005 EP 05008112
(43) Date of publication of application: 13.02.2008
(73) Proprietor: F.HOFFMANN-LA ROCHE AG, 4070 Basel (CH)
(72) Inventor: GEORGES, Guy, 82392 Habach (DE); GOLLER, Bernhard, 82377 Penzberg (DE); KRELL, Hans-Willi, 82377 Penzberg (DE); LIMBERG, Anja, 82377 Penzberg (DE); REIFF, Ulrike, 82377 Penzberg (DE); RUEGER, Petra, 82377 Penzberg (DE); RUETH, Matthias, 82377 Penzberg (DE)
(74) Representative: Schreiner, Siegfried
(86) International application number: PCT/EP2006/002477
(87) International publication number: WO 2006/108488

(56) References cited:
- WO-A-92/05173
- WO-A-03/035644
- WO-A-20/05007653
- D. BERGER ET AL.: "8-Anilinoimidazo[4,5-g]quinoline-7-carbon itriles as Src Kinase Inhibitors" BIOORG. MED. CHEM. LETT., vol. 12, 2002, pages 2761-2765, XP002334095 & WO 03/035065 A (AVENTIS PHARMACEUTICALS INC.) 1 May 2003 (2003-05-01)

## Description

The present invention relates to novel tricyclic azole derivatives, to a process for their manufacture, pharmaceutical compositions containing them and their manufacture as well as the use of these compounds as pharmaceutically active agents.

### Background of the Invention

Protein kinases regulate many different signaling processes by adding phosphate groups to proteins (Hunter, T., Cell 50 (1987) 823-829); particularly serine/threonine kinases phosphorylate proteins on the alcohol moiety of serine or threonine residues. The serine/threonine kinase family includes members that control cell growth, migration, differentiation, gene expression, muscle contraction, glucose metabolism, cellular protein synthesis, and regulation of the cell cycle.

The Aurora kinases are a family of serine/threonine kinases that are believed to play a key role in the protein phosphorylation events that are essential for the completion of essential mitotic events. The Aurora kinase family is made up of three key members: Aurora A, B and C (also known as Aurora-2, Aurora-1 and Aurora-3 respectively). Aurora-1 and Aurora-2 are described in US 6,207,401 of Sugen and in related patents and patent applications, e.g. EP 0 868 519 and EP 1051500.

For Aurora A there is increasing evidence that it is a novel proto-oncogene. Aurora A gene is amplified and transcript/protein is highly expressed in a majority of human tumor cell lines and primary colorectal, breast and other tumors. It has been shown that Aurora A overexpression leads to genetic instability shown by amplified centrosomes and significant increase in aneuploidy and transforms Rat1 fibroblasts and mouse NIH3T3 cells in vitro. Aurora A-transformed NIH3T3 cells grow as tumors in nude mice (Bischoff, J.R., and Plowman, G.D., Trends Cell Biol. 9 (1999) 454-459; Giet, R., and Prigent, C., J. Cell Sci. 112 (1999) 3591-3601; Nigg, E.A., Nat. Rev. Mol. Cell Biol. 2 (2001) 21-32; Adams, R.R., et al., Trends Cell Biol. 11 (2001) 49-54). Moreover, amplification of Aurora A is associated with aneuploidy and aggressive clinical behavior (Sen, S., et al., J. Natl.Cancer Inst. 94 (2002) 1320-1329) and amplification of its locus correlates with poor prognosis for patients with node-negative breast cancer (Isola, J.J., et al., Am. J. Pathology 147 (1995) 905-911). For these reasons it is proposed that Aurora A overexpression contributes to cancer phenotype by being involved in chromosome segregation and mitotic checkpoint control.

Human tumor cell lines depleted of Aurora A transcripts arrest in mitosis. Accordingly, the specific inhibition of Aurora kinase by selective inhibitors is recognized to stop uncontrolled proliferation, re-establish mitotic checkpoint control and lead to apoptosis of tumor cells. In a xenograft model, an Aurora inhibitor therefore slows tumor growth and induces regression (Harrington, E.A., et al., Nat. Med. 10 (2004) 262-267).

Low molecular weight inhibitors for protein kinases are widely known in the state of the art. For Aurora inhibition such inhibitors are based on i.e. quinazoline derivatives as claimed in the following patents and patent applications: WO 00/44728; WO 00/47212; WO 01/21594; WO 01/21595; WO 01/21596; WO 01/21597; WO 01/77085; WO 01/55116; WO 95/19169; WO 95/23141; WO 97/42187; WO 99/06396; pyrazole and triazole derivatives as claimed in the following patents and patent applications: WO 02/22601; WO 02/22602; WO 02/22603; WO 02/22604; WO 02/22605; WO 02/22606; WO 02/22607; WO 02/22608; WO 02/50065; WO 02/50066; WO 02/057259; WO 02/059112; WO 02/059111; WO 02/062789; WO 02/066461; WO 02/068415; pyrimidine derivatives: WO 03/077921; WO 03/078423; WO 03/078426; WO 03/078427; WO 04/000833 or imidazole, oxazole and thiazole derivatives: WO 02/96905; WO 04/005283.

JP 03/231687 relates to condensed pyrazole derivatives as neutrophin-inhibiting and analgetic agents. WO 03/035065 relates to benzimidazole derivatives as kinase inhibitors, especially as inhibitors against kinase insert domain containing receptor (KDR) tyrosine kinase, spleen tyrosine kinase (SYK) and inducible T cell kinase (ITK). And WO 2005/007653 refers to substituted 4,5,6,7-tetrahydropyrazolo[3,4-c]pyridines as kinase inhibitors, especially as inhibitors against tyrosine kinase with immunoglobulin and EGF (epidermal growth factor) repeats 2 (Tie 2) and KDR tyrosine kinase.

Some related tricyclic compounds are known as inhibitors of erythrocyte aggregation from US 4,835,280A and US 4,954,498A. Also Mertens, A., et al., J. Med. Chem. 30 (1987) 1279-1287; von der Saal, W., et al., J. Med. Chem. 32 (1989) 1481-1491; US 4,666,923A; US 4,695,567A and US 4,863,945A describe related tricycles as erythrocyte aggregation inhibitors. US 5,212,186A describes related tricycles for the treatment of cardiac insuffiency, hypertension and other diseases. WO 2005/111040 describes pyrrolobenzimidazolones with tubulin inhibitory activity as antiproliferative agents.

### Summary of the Invention

The present invention relates to tricyclic azole derivatives of the general formula I, 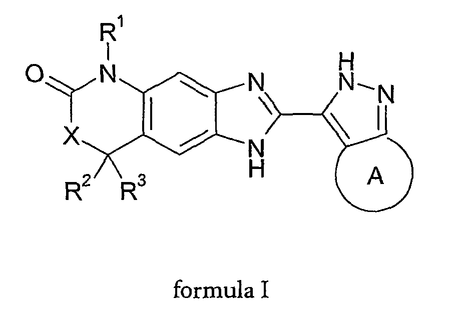 wherein,
- R¹: is hydrogen;
alkyl, alkenyl or alkynyl,
wherein said alkyl, alkenyl or alkynyl is optionally substituted one or several times by nitro, cyano or -Y-R⁴;
- Y: is a single bond, -C(O)NH-, -C(O)N(alkyl)-, -N(alkyl)C(O)-, -NHC(O)-, -NHC(O)NH-, -NHC(O)N(alkyl)-, -NHS(O)₂-, -S(O)₂NH-, -S(O)₂N(alkyl)-, -S(O)₂-, -S(O)-, -C(O)O-, -OC(O)-, -C(O)-, -P(O)(alkyl)-, -NH-, -N(alkyl)-, -O- or -S-;
- R⁴: is alkyl, wherein said alkyl is optionally substituted one or several times by halogen, hydroxy, alkoxy, alkoxyalkoxy, amino, alkylamino, dialkylamino, -C(O)OH or -C(O)NH₂; aryl, wherein the aryl is optionally substituted one or several times by halogen, cyano, nitro, amino, hydroxy, (C₁-C₄₎alkyl, (C₁-C₄)alkoxy, halogenated (C₁-C₄)alkyl or halogenated (C₁-C₄)alkoxy;
heteroaryl, wherein the heteroaryl is optionally substituted one or several times by alkyl;
cycloalkyl; or
heterocyclyl;
- R²: is hydrogen or alkyl;
- R³: is hydrogen or alkyl;
or alternatively R² and R³ form together with the carbon atom to which they are attached a (C₅-C₆)cycloalkyl ring;
- X: is a single bond, -CH₂- or -C(alkyl)₂-;
- ring A: is a 5 to 7 membered saturated ring optionally containing one or two heteroatoms independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-alkyl,
-C(O)-cycloalkyl,
-C(O)-heterocyclyl,
-C(O)-(CH₂)ₙ-phenyl, wherein the phenyl is optionally substituted once or twice with halogen, alkyl, alkoxy, nitro, amino, alkylamino, dialkylamino, cyano, trifluoromethyl or trifluoromethoxy,
-C(O)-(CH₂)ₙ-heteroaryl,
-C(O)-NH-phenyl, wherein the phenyl is optionally substituted once or twice with halogen, alkyl, alkoxy, nitro, amino, alkylamino, dialkylamino, cyano, trifluoromethyl or trifluoromethoxy, or
-S(O)₂-phenyl, wherein the phenyl is optionally substituted once or twice with halogen, alkyl, alkoxy, nitro, amino, alkylamino, dialkylamino, cyano, trifluoromethyl or trifluoromethoxy;
- n: is 0, 1 or 2;
and all pharmaceutically acceptable salts thereof.

The compounds according to this invention show activity as protein kinase inhibitors. Many diseases are associated with abnormal cellular responses triggered by protein kinase mediated events. These diseases include autoimmune diseases, inflammatory diseases, neurological and neurodegenerative diseases, cancer, cardiovascular diseases, allergies and asthma, Alzheimer's disease or hormone-related diseases. Accordingly, there has been a substantial effort in medicinal chemistry to find protein kinase inhibitors that are effective as therapeutic agents.

The compounds according to this invention in particular show activity as Aurora family kinase inhibitors, especially as Aurora A kinase inhibitors, and may therefore be useful for the treatment of diseases mediated by said kinase. Aurora A inhibition leads to cell cycle arrest in the G2 phase of the cell cycle and exerts an antiproliferative effect in tumor cell lines. This indicates that Aurora A inhibitors may be useful in the treatment of i.e. hyperproliferative diseases such as cancer and in particular colorectal, breast, lung, prostate, pancreatic, gastric, bladder, ovarian, melanoma, neuroblastoma, cervical, kidney or renal cancers, leukemias or lymphomas. Treatment of acute-myelogenous leukemia (AML, acute lymphocytic leukemia (ALL) and gastrointestinal stromal tumor (GIST) is included.

Objects of the present invention are the compounds of formula I and their tautomers, pharmaceutically acceptable salts, enantiomeric forms, diastereoisomers and racemates, their use as Aurora kinase inhibitors, the preparation of the above-mentioned compounds, medicaments containing them and their manufacture as well as the use of the above-mentioned compounds in treatment, control or prevention of illnesses, especially of illnesses and disorders as mentioned above like tumors or cancer (e.g. colorectal, breast, lung, prostate, pancreatic, gastric, bladder, ovarian, melanoma, neuroblastoma, cervical, kidney or renal cancers, leukemias or lymphomas) or in the manufacture of corresponding medicaments.

### Detailed Description of the Invention

The term "alkyl" as used herein means a saturated, straight-chain or branched-chain hydrocarbon containing from 1 to 6, preferably 1 to 4, carbon atoms, such as methyl, ethyl, n-propyl, isopropyl, n-butyl, 2-butyl, t-butyl.

The term "alkenyl" as used herein means an unsaturated straight-chain or branched aliphatic hydrocarbon group containing one double bond and having 2 to 6, preferably 2 to 4 carbon atoms. Examples of such "alkynyl group" are vinyl (ethenyl), allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "alkoxy" as used herein means an alkyl-O- group wherein the alkyl is defined as above.

The term "alkylamino" as used herein means an alkyl-NH- group wherein the alkyl is defined as above.

The term "dialkylamino" as used herein means an (alkyl)₂N- group wherein the alkyl is defined as above.

The term "alkyl, alkenyl, alkynyl, wherein said alkyl, alkenyl or alkynyl is optionally substituted one or several times by nitro, cyano or -Y-R⁴; " as used herein means an alkyl, alkenyl or alkynyl as defined above which is optionally substituted one to three times, preferably one to two times, especially one time by nitro, cyano or -Y-R⁴.

The term "alkyl, wherein the alkyl is optionally substituted one or several times" as used herein means an alkyl as defined above which is optionally substituted one to six times, preferably one to three times by halogen, preferably by fluorine or chlorine, especially by fluorine, or which is optionally substituted one to three times, preferably one to two times by hydroxy, alkoxy, alkoxyalkoxy, amino, alkylamino, dialkylamino, -C(O)OH or-C(O)NH2;. Examples of such optionally substituted alkyl groups are difluoromethyl, trifluoromethyl, 2,2,2-trifluoroethyl, perfluorethyl, difluoromethoxy, trifluoromethoxy, 2,2,2-trifluoroethoxy, perfluoroethoxy, 2-hydroxy-butyl, 2-hydroxy-ethyl, 2-hydroxy-propyl, 3-hydroxy-butyl, 2,3-dihydroxy-propyl, 2,3-dihydroxy-butyl, 1,2,3-trihydroxy-propyl, 2-hydroxy-pentyl, 2-methoxy-ethyl, 2-ethoxy-ethyl, 4-methoxy-butyl, 2-methoxy-butyl, 2-ethoxy-propyl, 3-propoxy-butyl, 2,3-dimethoxy-propyl, 2-ethoxy-3-methoxy-propyl, 2,3-diethoxy-butyl, 1,2,3-trimethoxy-propyl, 2-methoxy-pentyl, 2-(2-methoxy-ethoxy)-ethyl, 2-(2-ethoxy-ethoxy)-ethyl, 2-(2-propoxy-ethoxy)-ethyl, 3-(2-methoxy-ethoxy)-propyl, 3-(1-methoxy-ethoxy)-propyl, 4-(2-ethoxy-ethoxy)-butyl, 2-amino-butyl, 2-amino-ethyl, 2-amino-propyl, 3-amino-propyl, 3-amino-butyl, 2,3-diamino-propyl, 2-methylamino-butyl, 2-ethylamino-ethyl, 2-dimethylamino-ethyl, 2-dimethylamino-propyl, 3-diethylamino-propyl, 3-amino-butyl, 2,3-diamino-propyl, preferably 2,3-dihydroxy-propyl, 2-methoxy-ethyl, 2-(2-methoxy-ethoxy)-ethyl, trifluoromethyl, trifluoromethoxy.

The term "wherein the aryl is optionally substituted one or several times by" as used herein means that the aryl group in R⁴ is optionally substituted one to five times, preferably one to three times, especially one to two times.

The term "wherein the heteroaryl is optionally substituted one or several times by" as used herein means that the heteroaryl group in R⁴ is optionally substituted where possible one to two times, preferably one time.

The term "wherein said ring A can be optionally substituted one or several times" as used herein means that ring is optionally substituted where possible one to three times, preferably one to two times.

The term "halogenated alkyl," as used herein means an alkyl group as defined above which is substituted one or several times by halogen, preferably by fluorine or chlorine, especially fluorine. Examples are difluoromethyl, trifluoromethyl, 2,2,2-trifluoroethyl, perfluorethyl, and the like, especially trifluoromethyl.

The term "halogenated alkoxy" as used herein means an alkoxy group as defined above which is substituted one or several times by halogen, preferably by fluorine or chlorine, especially fluorine. Examples are difluoromethoxy, trifluoromethoxy, 2,2,2-trifluoroethoxy, perfluoroethoxy and the like, especially trifluoromethoxy.

The term "halogen" means fluorine, chlorine, bromine and iodine, preferably fluorine, chlorine or bromine and especially fluorine and chlorine.

The term "cycloalkyl" means a monocyclic saturated hydrocarbon ring with 3 to 7, preferably 3 to 6, ring atoms. Such saturated carbocyclic groups can be optionally substituted one or several times, preferably one to three times by alkyl, especially one to two times. Preferably such saturated carbocyclic groups are unsubstituted. Examples of such saturated carbocyclic groups are cyclopropyl, cyclobutyl, cyclopentyl, cyclohexyl, cycloheptyl, 3-methyl-cyclopentyl, 3,3-dimethyl-cyclohexyl, 3-methyl-cyclohexyl, 2-methyl-cyclohexyl, preferably cyclopropyl.

The cycloalkyl ring which is formed by R² and R³ together with the carbon atom to which they are attached is preferably a cyclopentyl or cyclohexyl ring, especially a cyclopentyl ring.

The cycloalkyl ring in the definition of the substituents of ring A is preferably a cyclopropyl, cyclobutyl or cyclopentyl ring, especially a cyclopropyl ring.

The term "heterocyclyl" means a saturated, monocyclic ring with 5 to 6 ring atoms which contains up to 3, preferably 1 or 2 heteroatoms selected independently from N, O or S and the remaining ring atoms being carbon atoms. Such saturated heterocyclic group can be optionally substituted one or several times, preferably one or two times a) by alkyl, preferably methyl, b) by -C(O)-alkyl, preferably acetyl, c) by oxo or d) by -S(O)₂-alkyl . Preferred substituents are a) alkyl or b) -C(O)-alkyl. Examples of such saturated heterocyclic groups include pyrrolidinyl, morpholinyl, piperazinyl, N-methyl-piperazinyl, N-acetyl-piperazinyl, piperazin-2-one, piperidyl and the like, preferably morpholino (4-morpholinyl).

The term "aryl" means a mono- or bicyclic aromatic ring with 6 to 10 ring carbon atoms. Examples of such aryl groups are phenyl and naphthyl, preferably phenyl.

The term "heteroaryl" means a mono- or bicyclic aromatic ring with 5 to 10, preferably 5 to 6, ring atoms, which contains up to 3, preferably 1 or 2 heteroatoms selected independently from N, O or S and the remaining ring atoms being carbon atoms. Examples of such heteroaryl groups include pyrrolyl, imidazolyl, pyrazolyl, triazolyl, tetrazolyl, furanyl, oxazolyl, isoxazolyl, thienyl, thiazolyl, pyridyl, pyrimidyl, pyridazinyl, pyrazinyl, indolyl, indazolyl, benzimidazolyl, benzothiophenyl, benzofuranyl, quinolyl, isoquinolyl, quinazolinyl and the like, especially pyridyl.

R¹ is hydrogen, alkyl, alkenyl or alkynyl, wherein said alkyl, alkenyl or alkynyl is optionally substituted one or several times by nitro, cyano or -Y-R⁴.

Y is a single bond, -C(O)NH-, -C(O)N(alkyl)-, -N(alkyl)C(O)-, -NHC(O)-, -NHC(O)NH-, -NHC(O)N(alkyl)-, -NHS(O)₂-, -S(O)₂NH-, -S(O)₂N(alkyl)-, -S(O)₂-, -S(O)-, -C(O)O-, -OC(O)-, -C(O)-, -P(O)(alkyl)-, -NH-, -N(alkyl)-, -O- or -S-, preferably a single bond.

R⁴ is alkyl, wherein said alkyl is optionally substituted one or several times by halogen, hydroxy, alkoxy, alkoxyalkoxy, amino, alkylamino, dialkylamino, -C(O)OH or -C(O)NH₂; aryl, wherein the aryl is optionally substituted one or several times by halogen, cyano, nitro, amino, hydroxy, (C₁-C₄)alkyl, (C₁-C₄)alkoxy, halogenated (C₁-C₄)alkyl or halogenated (C₁-C₄)alkoxy; heteroaryl, wherein the heteroaryl is optionally substituted one or several times by alkyl; cycloalkyl; or heterocyclyl. Preferably R⁴ is heterocyclyl, especially morpholino.

R² is hydrogen or alkyl, preferably alkyl; R³ is hydrogen or alkyl, preferably alkyl; or alternatively R² and R³ form together with the carbon atom to which they are attached a (C₅-C₆)cycloalkyl ring. Preferably R² and R³ are both alkyl.

X is a single bond, -CH₂- or -C(alkyl)₂-, preferably a single bond.

Ring A is a 5 to 7 membered, preferably 5 to 6 membered, saturated ring optionally containing one or two, preferably one, heteroatoms independently selected from oxygen, nitrogen or sulfur and the remaining ring atoms being carbon atoms. If ring A are contains two heteroatoms independently selected from oxygen, nitrogen or sulfur, said heteroatoms are not adjacent. Said ring A can be optionally substituted, where possible, one or several times, preferably one to three times, especially one to two times by alkyl. Examples of such ring A include cyclopentyl, cyclohexyl, cycloheptyl, methyl-cyclopentyl, methyl-cyclohexyl, 1,1-dimethyl-cyclohexyl, 1,3-dimethyl-cyclohexyl, 1,4-dimethyl-cyclohexyl, cycloheptyl, tetrahydrofuran, pyrrolidine, N-methyl-pyrrolidine, N-ethyl-pyrrolidine, N-isopropyl-pyrrolidine, tetrahydrothiophene, [1,3]dioxolane, tetrahydropyran, piperidine, N-methyl-piperidine, N-ethyl-piperidine, N-isopropyl-piperidine, tetrahydrothiopyran, azepane, [1,3]dioxane, [1,3]dioxepane and [1,4]dithiepane, preferably cyclopentyl, cyclohexyl, pyrrolidine, tetrahydrothiophene, tetrahydropyran, piperidine and tetrahydrothiopyran.

If said ring A contains one nitrogen, said nitrogen can be optionally substituted once, by -CH₂-phenyl, -C(O)-alkyl, -C(O)-cycloalkyl, -C(O)-heterocyclyl, -C(O)-(CH₂)ₙ-phenyl, wherein the phenyl is optionally substituted once or twice with halogen, alkyl, alkoxy, nitro, amino, alkylamino, dialkylamino, cyano, trifluoromethyl or trifluoromethoxy, -C(O)-(CH₂)ₙ-heteroaryl, -C(O)-NH-phenyl, wherein the phenyl is optionally substituted once or twice with halogen, alkyl, alkoxy, nitro, amino, alkylamino, dialkylamino, cyano, trifluoromethyl or trifluoromethoxy, or -S(O)₂-phenyl, wherein the phenyl is optionally substituted once or twice with halogen, alkyl, alkoxy, nitro, amino, alkylamino, dialkylamino, cyano, trifluoromethyl or trifluoromethoxy. Said nitrogen-containing ring A is preferably substituted by -CH₂-phenyl, -C(O)-cycloalkyl (preferably -C(O)-cyclopropyl) -C(O)-heterocyclyl (preferably -C(O)-morpholino), -C(O)-(CH₂)ₙ-phenyl, wherein the phenyl is optionally substituted once or twice with halogen ( preferably fluorine), -C(O)-(CH₂)ₙ-heteroaryl (preferably -C(O)-(CH₂)ₙ-thienyl), -C(O)-NH-phenyl, wherein the phenyl is optionally substituted once or twice with alkyl, or -S(O)₂-phenyl, wherein the phenyl is optionally substituted once or twice with halogen (preferably fluorine). Examples of such substituted nitrogen-containing ring A include e.g. N-benzyl-pyrrolidine, N-acetyl-pyrrolidine, N-(4-fluoro-benzenesulfonyl)-pyrrolidine, N-(morpholine-4-carbonyl)-piperidine, N-[(2,6-diethyl-phenyl)-aminocarbonyl]-piperidine, N-(2-thiophen-2-yl-acetyl)-piperidine and the like.

n is 0,1 or 2, preferably 0 or 1.

The ring system formed by fusion of ring A with the pyrazole is a 8 to 10, preferably 8 to 9, membered bicyclic ring system (2,4,5,6-tetrahydro-cyclopentapyrazole or the tautomeric form 2,4,5,6-tetrahydro-cyclopentapyrazole; 4,5,6,7-tetrahydro-2H-indazole or the tautomeric form 4,5,6,7-tetrahydro-1H-indazole; 2,4,5,6,7,8-hexahydro-cycloheptapyrazole or the tautomeric form 1,4,5,6,7,8-hexahydro-cycloheptapyrazole) wherein one or two, preferably one, carbon atoms of ring A (excluding the bridge atoms) can be optionally replaced by a heteroatom independently selected from oxygen, nitrogen or sulfur. If two carbon atoms of ring A are replaced by a heteroatom independently selected from oxygen, nitrogen or sulfur, said heteroatoms are not adjacent.

Examples of such ring systems formed by fusion of ring A with the pyrazole include 2,4,5,6-tetrahydro-cyclopentapyrazole, 4,5,6,7-tetrahydro-2H-indazole, 2,4,5,6,7,8-hexahydro-cycloheptapyrazole, 2,6-dihydro-4H-furo[3,4-c]pyrazole, 2,4,5,6-tetrahydro-pyrrolo[3,4-c]pyrazole, 2,4,5,6-tetrahydro-pyrrolo[2,3-c]pyrazole, 2,6-dihydro-4H-thieno[3,4-c]pyrazole, 2H-[1,3]dioxolo[4,5-c]pyrazole, 2,4,6,7-tetrahydro-pyrano[4,3-c]pyrazole, 2,4,5,7-tetrahydro-pyrano[3,4-c]pyrazole, 4,5,6,7-tetrahydro-1*H*-pyrazolo[4,3-c]pyridine, 2,4,6,7-tetrahydro-1*H-*pyrazolo[4,3-c]pyridine, 2,4,6,7-tetrahydro-thiopyrano[4,3-c]pyrazole, 2,4,5,7-tetrahydro-thiopyrano[3,4-c]pyrazole, 2,4-dihydro-[1,3]dioxino[4,5-c]pyrazole, 2,7-dihydro-4,6-dioxa-1,2-diaza-indene, 4,5-dihydro-2H-6,8-dioxa-1,2-diaza-azulene and 6,7-dihydro-2H,5H-4,8-dithia-1,2-diaza-azulene, preferably 2,4,5,6-tetrahydro-cyclopentapyrazole, 4,5,6,7-tetrahydro-2H-indazole, 2,4,5,6-tetrahydro-pyrrolo[3,4-c]pyrazole, 2,6-dihydro-4H-thieno[3,4-c]pyrazole, 2,4,6,7-tetrahydro-pyrano[4,3-c]pyrazole, 4,5,6,7-tetrahydro-1*H*-pyrazolo[4,3-c]pyridine and 2,4,6,7-tetrahydro-thiopyrano [4,3-c] pyrazole.

If Y is -NH- or -N(alkyl)-, and R⁴ is alkyl, then said alkyl is a substituted one and the substituents are selected from halogen, hydroxy, alkoxy, alkoxyalkoxy, amino, alkylamino, dialkylamino, -C(O)OH or -C(O)NH₂.

If Y is -O-, and R⁴ is alkyl, then said alkyl is a substituted one and the substituents are selected from halogen, hydroxy, alkoxyalkoxy, amino, alkylamino, dialkylamino, -C(O)OH or -C(O)NH₂.

As used herein, in relation to mass spectrometry (MS) the term "API+" refers to positive atmospheric pressure ionization mode.

As used herein, in relation to nuclear magnetic resonance (NMR) the term "D₆-DMSO" refers to deuterated dimethylsulfoxide.

The compounds of formula I can exist in different tautomeric forms and in variable mixtures thereof. All tautomeric forms of the compounds of formula I and mixtures thereof are an objective of the invention. For example, the imidazole part of the tricyclic ring system of formula I can exist in two tautomeric forms as shown here below:

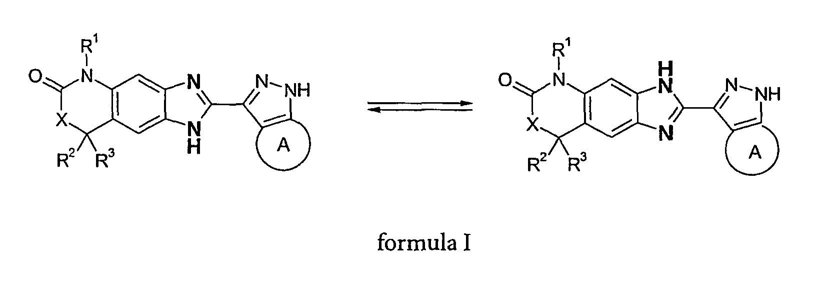

Also, e.g. the pyrazole ring of formula I can form two tautomeric forms as shown here below:

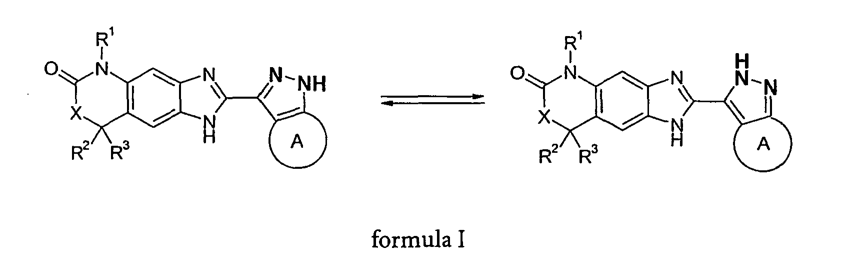

An embodiment of the invention are the compounds according to formula I, wherein
- ring A: is a 5 to 7 membered saturated ring optionally containing one or two heteroatoms independently selected from oxygen, nitrogen or sulfur and the remaining ring atoms being carbon atoms,
wherein said ring A can be optionally substituted one or several times by alkyl.

Another embodiment of the invention are the compounds according to formula I, wherein
- R¹: is hydrogen or alkyl,
wherein said alkyl is optionally substituted once by -Y-R⁴;
- Y: is a single bond;
- R⁴: is heterocyclyl, preferably morpholino;
- R²: is alkyl;
- R³: is alkyl;
- X: is a single bond;
- ring A: is a 5 to 7 membered, preferably 5 to 6 membered, saturated ring optionally containing one or two heteroatoms independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-cycloalkyl, preferably -C(O)-cyclopropyl,
-C(O)-heterocyclyl, preferably -C(O)-morpholino,
-C(O)- (CH₂)ₙ-phenyl, wherein the phenyl is optionally substituted once or twice with halogen, preferably fluorine, -C(O)-(CH₂)ₙ-heteroaryl preferably -C(O)-(CH₂)ₙ-thienyl, -C(O)-NH-phenyl, wherein the phenyl is optionally substituted once or twice with alkyl, or
-S(O)₂-phenyl, wherein the phenyl is optionally substituted once or twice with halogen, preferably fluorine; and
- n: is 0 or 1.

Another embodiment of the invention are the compounds according to formula I, wherein
- R¹, R² and R³: are alkyl;
- X: is a single bond;
- ring A: is a 5 to 7 membered, preferably 5 to 6 membered, saturated ring optionally containing one heteroatom independently selected from oxygen, nitrogen or sulfur and the remaining ring atoms being carbon atoms,
wherein said ring A can be optionally substituted one or several times by alkyl,
and if said ring A contains one nitrogen, said nitrogen can be
optionally substituted once by
-CH₂-phenyl,
-C(O)-cycloalkyl,
-C(O)-heterocyclyl,
-C(O)-(CH₂)ₙ-phenyl, wherein the phenyl is optionally substituted once or twice with halogen,
-C(O)-(CH₂)ₙ-thienyl,
-C(O)-NH-phenyl, wherein the phenyl is optionally substituted once or twice with alkyl, or
-S(O)₂-phenyl, wherein the phenyl is optionally substituted once or twice with halogen; and
- n: is 0 or 1.

Another embodiment of the invention are the compounds according to formula I, wherein
- ring A: is a 5 to 7 membered saturated ring optionally containing one heteroatom independently selected from oxygen, nitrogen or sulfur and the remaining ring atoms being carbon atoms, wherein said ring A can be optionally substituted one or several times by alkyl.

Another embodiment of the invention are the compounds according to formula I, wherein
- R¹, R² and R³: are alkyl;
- X: is a single bond; and
- ring A: is a 5 to 7 membered saturated ring optionally containing one heteroatom independently selected from oxygen, nitrogen or sulfur and the remaining ring atoms being carbon atoms, wherein said ring A can be optionally substituted one or several times by alkyl.

Another embodiment of the invention are the compounds according to formula I, wherein
- R⁴: is heterocyclyl.

Another embodiment of the invention are the compounds according to formula I, wherein
- R⁴: is heterocyclyl; and
- ring A: is a 5 to 7 membered saturated ring optionally containing one heteroatom independently selected from oxygen, nitrogen or sulfur and the remaining ring atoms being carbon atoms, wherein said ring A can be optionally substituted one or several times by alkyl.

Another embodiment of the invention are the compounds according to formula I, wherein
- X: is a single bond.

Another embodiment of the invention are the compounds according to formula I, wherein
- X: is a single bond; and
- ring A: is a 5 to 7 membered saturated ring optionally containing one heteroatom independently selected from oxygen, nitrogen or sulfur and the remaining ring atoms being carbon atoms, wherein said ring A can be optionally substituted one or several times by alkyl.

Another embodiment of the invention are the compounds according to formula I, wherein
- ring A: is a 5 to 7 membered saturated hydrocarbon ring.

Another embodiment of the invention are the compounds according to formula I, wherein
- X: is a single bond; and
- ring A: is a 5 to 7 membered, preferably 5 to 6 membered, saturated hydrocarbon ring.

Another embodiment of the invention are the compounds according to formula I, wherein
- R¹: is hydrogen or alkyl,
wherein said alkyl is optionally substituted once by -Y-R⁴;
- Y: is a single bond;
- R⁴: is heterocyclyl, preferably morpholino;
- R²: is alkyl;
- R³: is alkyl;
- X: is a single bond; and
- ring A: is a 5 to 7 membered, preferably 5 to 6 membered, saturated hydrocarbon ring.

Such compounds, for example, may be selected from the group consisting of:
7,7-Dimethyl-2-(1,4,5,6-tetrahydro-cyclopentapyrazol-3-yl)-5,7-dihydro-3H-imidazo[4,5-f]indol-6-one;
7,7-Dimethyl-2-(4,5,6,7-tetrahydro-1H-indazol-3-yl)-5,7-dihydro-3H-imidazo [4,5-f] indol-6-one;
5,7,7-Trimethyl-2-(1,4,5,6-tetrahydro-cyclopentapyrazol-3-yl)-5,7-dihydro-3H-imidazo[4,5-f]indol-6-one;
5-Ethyl-7,7-dimethyl-2-(1,4,5,6-tetrahydro-cyclopentapyrazol-3-yl)-5,7-dihydro-3H-imidazo [4,5-f] indol-6-one;
7,7-Dimethyl-5-propyl-2-(1,4,5,6-tetrahydro-cyclopentapyrazol-3-yl)-5,7-dihydro-3H-imidazo[4,5-f]indol-6-one;
5-Isopropyl-7,7-dimethyl-2-(1,4,5,6-tetrahydro-cyclopentapyrazol-3-yl)-5,7-dihydro-3H-imidazo[4,5-f]indol-6-one; and
7,7-Dimethyl-5-(3-morpholin-4-yl-propyl)-2-(1,4,5,6-tetrahydro-cyclopentapyrazol-3-yl)-5,7-dihydro-3H-imidazo[4,5-f]indol-6-one;
5-Ethyl-7,7-dimethyl-2-(4,5,6,7-tetrahydro-1H-indazol-3-yl)-5,7-dihydro-3H-imidazo[4,5-f]indol-6-one;
5-Isopropyl-7,7-dimethyl-2-(4,5,6,7-tetrahydro-1H-indazol-3-yl)-5,7-dihydro-3H-imidazo[4,5-f]indol-6-one;
7,7-Dimethyl-5-(3-morpholin-4-yl-propyl)-2-(4,5,6,7-tetrahydro-1H-indazol-3-yl)-5,7-dihydro-3H-imidazo[4,5-f]indol-6-one; and
5,7,7-Triethyl-2-(4,5,6,7-tetrahydro-1*H*-indazol-3-yl)-5,7-dihydro-3*H-*imidazo[4,5-*f*] indol-6-one.

Another embodiment of the invention are the compounds according to formula I, wherein
- R¹, R² and R³: are alkyl;
- X: is a single bond; and
- ring A: is a 5 to 7 membered, preferably 5 to 6 membered, saturated ring containing one heteroatom independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-cycloalkyl, preferably -C(O)-cyclopropyl,
-C(O)-heterocyclyl, preferably -C(O)-morpholino,
-C(O)-(CH₂)ₙ-phenyl, wherein the phenyl is optionally substituted once or twice with halogen, preferably fluorine,
-C(O)-(CH₂)ₙ-thienyl,
-C(O)-NH-phenyl, wherein the phenyl is optionally substituted once or twice with alkyl, or
-S(O)₂-phenyl, wherein the phenyl is optionally substituted once or twice with halogen, preferably fluorine; and
- n: is 0 or 1;

Such compounds, for example, may be selected from the group consisting of:
5-Ethyl-7,7-dimethyl-2-(1,4,6,7-tetrahydro-pyrano[4,3-c]pyrazol-3-yl)-5,7-dihydro-3H-imidazo [4,5-f] indol-6-one;
2-(5-Benzyl-4,5,6,7-tetrahydro-1*H*-pyrazolo[4,3-*c*]pyridin-3-yl)-5-ethyl-7,7-dimethyl-5,7-dihydro-3*H*-imidazo[4,5-*f*]indol-6-one;
5-Ethyl-7,7-dimethyl-2-(4,5,6,7-tetrahydro-1*H*-pyrazolo[4,3-*c*]pyridin-3-yl)-5,7-dihydro-3*H*-imidazo[4,5-*f*]indol-6-one;
2-(5-Benzyl-1,4,5,6-tetrahydro-pyrrolo[3,4-*c*]pyrazol-3-yl)-5-ethyl-7,7-dimethyl-5,7-dihydro-3*H*-imidazo[4,5-*f*]indol-6-one;
5-Ethyl-7,7-dimethyl-2-(1,4,6,7-tetrahydro-thiopyrano[4,3-*c*]pyrazol-3-yl)-5,7-dihydro-3*H*-imidazo[4,5-*f*]indol-6-one;
2-(5-Cyclopropanecarbonyl-1,4,5,6-tetrahydro-pyrrolo[3,4-*c*]pyrazol-3-yl)-5-ethyl-7,7-dimethyl-5,7-dihydro-3*H*-imidazo[4,5-*f*]indol-6-one;
5-Ethyl-2-{5-[2-(4-fluoro-phenyl)-acetyl]-4,5,6,7-tetrahydro-1*H*-pyrazolo[4,3-*c*]pyridin-3-yl}-7,7-dimethyl-5,7-dihydro-3*H*-imidazo[4,5-*f*]indol-6-one;
5-Ethyl-2-[5-(4-fluoro-benzenesulfonyl)-4,5,6,7-tetrahydro-1*H*-pyrazolo[4,3-*c*]pyridin-3-yl]-7,7-dimethyl-5,7-dihydro-3*H*-imidazo[4,5-*f*]indol-6-one;
5-Ethyl-7,7-dimethyl-2-[5-(morpholine-4-carbonyl)-4,5,6,7-tetrahydro-1*H-*pyrazolo[4,3-*c*]pyridin-3-yl]-5,7-dihydro-3*H*-imidazo[4,5-*f*]indol-6-one;
5-Ethyl-2-[5-(4-fluoro-benzoyl)-4,5,6,7-tetrahydro-1*H*-pyrazolo[4,3-*c*]pyridin-3-yl]-7,7-dimethyl-5,7-dihydro-3*H*-imidazo[4,5-*f*]indol-6-one;
2-(4,6-Dihydro-1*H*-thieno[3,4-*c*]pyrazol-3-yl)-5-ethyl-7,7-dimethyl-5,7-dihydro-3*H*-imidazo[4,5-*f*]indol-6-one;
5-Ethyl-7,7-dimethyl-2-[5-(2-thiophen-2-yl-acetyl)-1,4,5,6-tetrahydro-pyrrolo[3,4-*c*]pyrazol-3-yl]-5,7-dihydro-3*H*-imidazo[4,5-*f*]indol-6-one;
2-(4,6-Dihydro-1*H*-thieno[3,4-*c*]pyrazol-3-yl)-5-isopropyl-7,7-dimethyl-5,7-dihydro-3*H*-imidazo[4,5-*f*]indol-6-one;
3-(5-Ethyl-7,7-dimethyl-6-oxo-3,5,6,7-tetrahydro-imidazo[4,5-*f*]indol-2-yl)-4,6-dihydro-1*H*-pyrrolo[3,4-*c*]pyrazole-5-carboxylic acid (2,6-diethyl-phenyl)-amide;
5-Ethyl-2-[5-(4-fluoro-benzenesulfonyl)-1,4,5,6-tetrahydro-pyrrolo[3,4-*c*]pyrazol-3-yl]-7,7-dimethyl-5,7-dihydro-3*H*-imidazo[4,5-*f*]indol-6-one;
3-(5-Isopropyl-7,7-dimethyl-6-oxo-3,5,6,7-tetrahydro-imidazo[4,5-*f*]indol-2-yl)-4,6-dihydro-1*H*-pyrrolo[3,4-*c*]pyrazole-5-carboxylic acid (2,6-diethyl-phenyl)-amide; and
5-Isopropyl-7,7-dimethyl-2-[5-(2-thiophen-2-yl-acetyl)-1,4,5,6-tetrahydro-pyrrolo[3,4-*c*]pyrazol-3-yl]-5,7-dihydro-3*H*-imidazo[4,5-*f*]indol-6-one.

Another embodiment of the invention are the compounds according to formula I, wherein
- R¹, R² and R³: are alkyl;
- X: is a single bond; and
- ring A: is a 5 to 7 membered, preferably 5 to 6 membered, saturated ring containing one heteroatom independently selected from oxygen, nitrogen or sulfur and the remaining ring atoms being carbon atoms.

Another embodiment of the invention are the compounds according to formula I, wherein
- R¹, R² and R³: are alkyl;
- X: is a single bond; and
- ring A: is a 5 to 7 membered, preferably 5 to 6 membered, saturated ring containing one nitrogen and the remaining ring atoms being carbon atoms,
wherein said nitrogen is substituted once by
-CH₂-phenyl,
-C(O)-alkyl,
-C(O)-cycloalkyl,
-C(O)-heteroryclyl,
-C(O)-(CH₂)ₙ-phenyl, wherein the phenyl is optionally substituted once or twice with halogen, alkyl, alkoxy, nitro, amino, alkylamino, dialkylamino, cyano, trifluoromethyl or trifluoromethoxy,
-C(O)-(CH₂)ₙ-heteroaryl,
-C(O)-NH-phenyl, wherein the phenyl is optionally substituted once or twice with halogen, alkyl, alkoxy, nitro, amino, alkylamino, dialkylamino, cyano, trifluoromethyl or trifluoromethoxy, or
-S(O)₂-phenyl, wherein the phenyl is optionally substituted once or twice with halogen, alkyl, alkoxy, nitro, amino, alkylamino, dialkylamino, cyano, trifluoromethyl or trifluoromethoxy; and
- n: is 0, 1 or 2.

Another embodiment of the invention are the compounds according to formula I, wherein
- R¹, R² and R³: are alkyl;
- X: is a single bond; and
- ring A: is a 5 to 7 membered, preferably 5 to 6 membered, saturated ring containing one nitrogen and the remaining ring atoms being carbon atoms,
wherein said nitrogen is substituted once by
-CH₂-phenyl,
-C(O)-cycloalkyl, preferably -C(O)-cyclopropyl,
-C(O)-heterocyclyl, preferably -C(O)-morpholino,
-C(O)-(CH₂)ₙ-phenyl, wherein the phenyl is optionally substituted once or twice with halogen, preferably fluorine,
-C(O)-(CH₂)ₙ-thienyl,
-C(O)-NH-phenyl, wherein the phenyl is optionally substituted once or twice with alkyl, or
-S(O)₂-phenyl, wherein the phenyl is optionally substituted once or twice with halogen, preferably fluorine; and
- n: is 0 or 1.

Another embodiment of the invention is a process for the preparation of the compounds of formula I comprising the steps of
a) reacting a compounds of formula II, 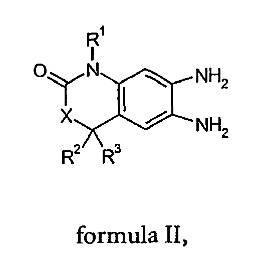 wherein X, R¹, R² and R³ have the significance given above for formula I,
   with a compound of formula III, 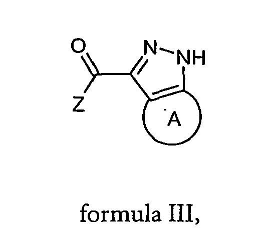 wherein ring A has the significance given above for formula I and Z is -OH, -Cl, -H, -OMe or hydroxybenzotriazole,
   to give the compounds of formula I 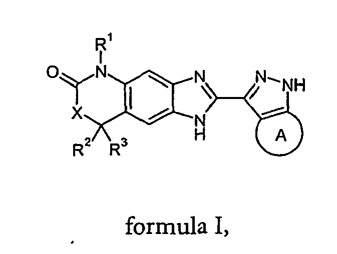
b) isolating the compounds of formula I; and
c) if desired, converting the compounds of formula I into their pharmaceutically acceptable salts.

The tricyclic compounds of formula I, or a pharmaceutically acceptable salt thereof, which are subject of the present invention, may be prepared by any process known to be applicable to the preparation of chemically-related compounds. Such processes, when used to prepare a compound of the formula I, or a pharmaceutically-acceptable salt thereof, are illustrated by the following representative schemes 1 to 3 and examples in which, unless otherwise stated, R¹, R², R³, R⁴, X, Y and ring A have the significance given herein before for formula I. Necessary starting materials are either commercially available or they may be obtained by standard procedures of organic chemistry. The preparation of such starting materials is described within the accompanying examples or in the literature cited below with respect to scheme 1 to 3. Alternatively necessary starting materials are obtainable by analogous procedures to those illustrated which are within the ordinary skill of an organic chemist.

The tricyclic fused imidazole ring of formula I can be formed by different synthetic pathways in analogy to methods described in the literature (Mertens, A., et al., J. Med. Chem. 30 (1987) 1279-1287; US 4,695,567A).

One route for the preparation of compounds of formula I (Scheme 1) starts from diamines of formula II which can be reacted with carboxylic acids (compounds of formula III wherein Z is -OH), acid chlorides (compounds of formula III wherein Z is -Cl), aldehydes (compounds of formula III wherein Z is -H), methyl carboxylates (compounds of formula III wherein Z is -OMe) or activated esters (compounds of formula III wherein A is e.g. hydroxybenzotriazole). For detailed procedures see Mertens, A., et al., J. Med. Chem. 30 (1987) 1279-1287 and US 4,695,567A.

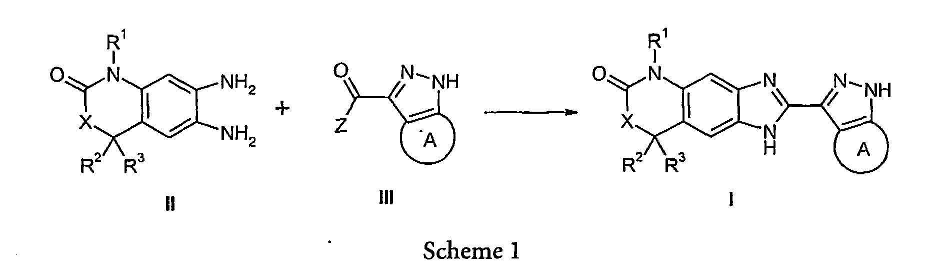

In scheme 1, A, R¹, R², R³ and X have the significance as given above for formula I and Z is -OH, -Cl, -H, -OMe or e.g. hydroxybenzotriazole.

The synthesis of diamines of formula II or precursors thereof is described in Mertens, A., et al., J. Med. Chem. 30 (1987) 1279-1287; von der Saal, W., et al., J. Med. Chem. 32 (1989) 1481-1491; US 4,666,923A, US 4,695,567A, US 4,863,945A and US 4,985,448A. For instance, the diamines of formula II, wherein A is a single bond are named IIa and can be synthesized according to US 4,666,923A, DE 34 10 168 and Mertens, A., et al., J. Med. Chem. 30 (1987) 1279-1287 as shown in Scheme 2a:

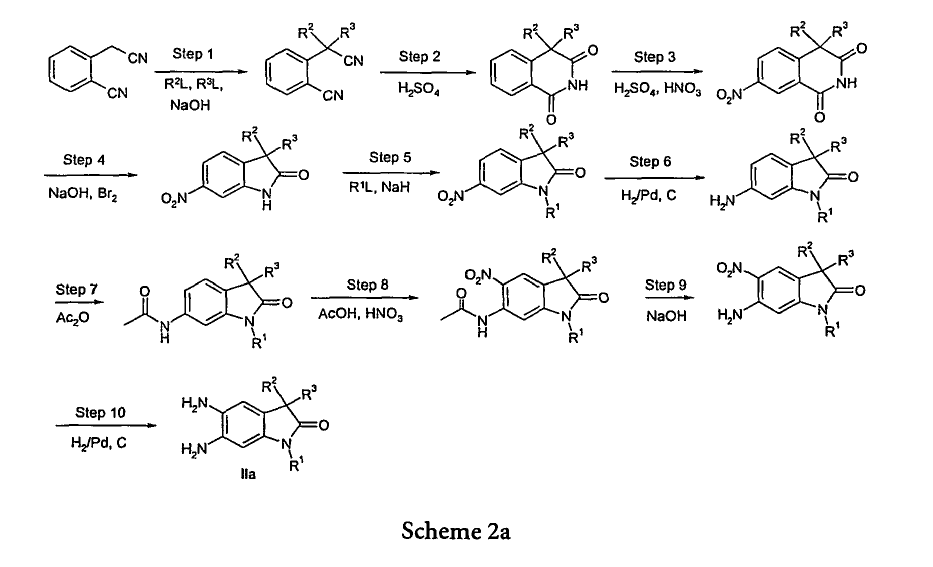

In scheme 2a, R¹, R² and R³ have the significance as given above for formula I and L represents a leaving group as e.g. iodine, bromine, chlorine, triflate and the like.

In an alternative procedure diamines of formula IIa can be obtained by an alkylation of diamines of formula IIb (compounds II wherein A is a single bond and R¹ is hydrogen) as shown in scheme 2b.

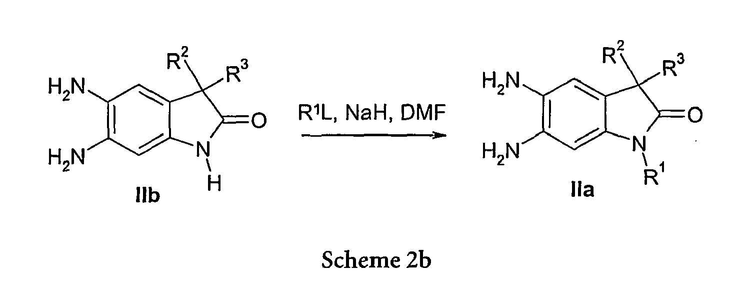

Diamines of formula IIb can be synthesized according to scheme 1 under omission of step 5.

Annelated pyrazoles of formula III in scheme 1 are either commercially available or they can be prepared by different synthetic routes according to the nature of "Z" and "ring A". If "Z" is hydroxy the corresponding annelated pyrazole 3-carboxylic acids are named IIIa and can be manufactured e.g. as shown in the following scheme 3.

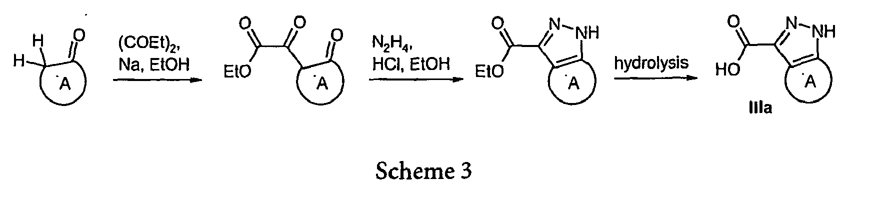

In scheme 3, A has the significance as given above for formula I. Various cyclic carbonyl compounds with an α-methylene group undergo mixed Claisen condensation with diethyl oxalate. The resulting α,γ-diketo esters can be condensed with hydrazine to give the annelated pyrazole. After hydrolysis of the ester functionality the desired annelated pyrazole 3-carboxylic acids IIIa are obtained (see e.g. van Herk, T., et al., J. Med. Chem. 46 (2003) 3945-3951).

The preparation compounds of formula III wherein Z is -OEt and A is a 5 to 7 membered saturated ring containing one nitrogen atom wherein the nitrogen is substituted by methyl (especially 4-methyl-1,4,5,6-tetrahydro-pyrrolo[3,2-c]pyrazole-3-carboxylic acid ethyl ester, 4-methyl-4,5,6,7-tetrahydro-1H-pyrazolo[4,3-b]pyridine-3-carboxylic acid ethyl ester and 4-methyl-1,4,5,6,7,8-hexahydro-1,2,4-triaza-azulene-3-carboxylic acid ethyl ester) are described in Möhrle, H., et al., Chem. Ber. 119 (1986) 3591-3599, starting from the corresponding lactame acetals by reaction with diazo-acetic acid ester.

Certain substituents on the groups R¹ may not be inert to the conditions of the synthesis sequences described above and may require protection by standard protecting groups known in the art. For instance, an amino or hydroxyl group may be protected as an acetyl or tert.-butoxycarbonyl derivative. Alternatively, some substituents may be derived from others at the end of the reaction sequence. For instance, a compound of formula I may be synthesized bearing a nitro-, an ethoxycarbonyl, a sulfonic acid substituent on the group R¹, which substituents are finally converted to an amino-, alkylamino-, dialkylamino-, acylamino-, alkylsulfonylamino, arylsulfonylamino substituent, or to a carboxamide substituent, or to a sulfonamide substituent by standard procedures.

Medicaments containing a compound of the present invention or a pharmaceutically acceptable salt thereof and a therapeutically inert carrier are an object of the present invention, as is a process for their production, which comprises bringing one or more compounds of the present invention and/or pharmaceutically acceptable salts and, if desired, one or more other therapeutically valuable substances into a galenical administration form together with one or more therapeutically inert carriers.

In accordance with the invention the compounds of the present invention as well as their pharmaceutically acceptable salts are useful in the control or prevention of illnesses. Based on their Aurora tyrosine kinases inhibition and their antiproliferative activity, said compounds are useful for the treatment of diseases such as cancer in humans or animals and for the production of corresponding medicaments. The dosage depends on various factors such as manner of administration, species, age and/or individual state of health.

An embodiment of the invention is a pharmaceutical composition, containing one or more compounds according to formula I, together with pharmaceutically acceptable excipients.

Another embodiment of the invention is a pharmaceutical composition containing one or more compounds of formula I as active ingredients together with pharmaceutically acceptable adjuvants for the treatment of diseases mediated by an inappropriate activation of Aurora family tyrosine kinases.

Another embodiment of the invention is a pharmaceutical composition, containing one or more compounds according to formula I, for the inhibition of tumor growth.

Another embodiment of the invention is a pharmaceutical composition containing one or more compounds of formula I as active ingredients together with pharmaceutically acceptable adjuvants for the treatment of colorectal, breast, lung, prostate, pancreatic, gastric, bladder, ovarian, melanoma, neuroblastoma, cervical, kidney or renal cancers, leukemias or lymphomas.

Another embodiment of the invention is a pharmaceutical composition containing one or more compounds of formula I as active ingredients together with pharmaceutically acceptable adjuvants for the treatment of acute-myelogenous leukemia (AML, acute lymphocytic leukemia (ALL) and gastrointestinal stromal tumor (GIST).

Another embodiment of the invention is the use of one or more compounds of formula I for the manufacture of medicaments for the treatment of diseases mediated by an inappropriate activation of Aurora family tyrosine kinases.

Another embodiment of the invention is the use of a compound according to formula I, for the manufacture of corresponding medicaments for the inhibition of tumor growth.

Another embodiment of the invention is the use of a compound according to formula I, for the manufacture of corresponding medicaments for the treatment of colorectal, breast, lung, prostate, pancreatic, gastric, bladder, ovarian, melanoma, neuroblastoma, cervical, kidney or renal cancers, leukemias or lymphomas.

Another embodiment of the invention is the use of a compound according to formula I, for the manufacture of medicaments for the treatment of acute-myelogenous leukemia (AML, acute lymphocytic leukemia (ALL) and gastrointestinal stromal tumor (GIST).

Another embodiment of the invention is the use of the compounds of formula I as Aurora A tyrosine kinase inhibitors.

Another embodiment of the invention is the use of the compounds of formula I as anti-proliferating agents.

Another embodiment of the invention is the use of one or more compounds of formula I for the treatment of cancer.

The compounds according to the present invention may exist in the form of their pharmaceutically acceptable salts. The term "pharmaceutically acceptable salt" refers to conventional acid-addition salts that retain the biological effectiveness and properties of the compounds of formula I and are formed from suitable non-toxic organic or inorganic acids. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, naphthalenesulfonic acid, naphthalenedisulfonic acid, methanesulfonic acid, ethanesulfonic acid and the like. The chemical modification of a pharmaceutical compound (i.e. a drug) into a salt is a technique well known to pharmaceutical chemists to obtain improved physical and chemical stability, hygroscopicity, flowability and solubility of compounds. See, e.g. Bastin, R.J., et al, Organic Proc. Res. Dev. 4 (2000) 427-435.

The compounds of formula I can contain one or several chiral centers and can then be present in a racemic or in an optically active form. The racemates can be separated according to known methods into the enantiomers. For instance, diastereomeric salts which can be separated by crystallization are formed from the racemic mixtures by reaction with an optically active acid such as e.g. D- or L-camphorsulfonic acid. Alternatively separation of the enantiomers can also be achieved by using chromatography on chiral HPLC-phases (HPLC: High Performance Liquid Chromatography) which are commercially available.

### Pharmacological activity

The compounds of formula I and their pharmaceutically acceptable salts possess valuable pharmacological properties. It has been found that said compounds show activity as inhibitors of the Aurora kinase family and also show anti-proliferative activity. Consequently the compounds of the present invention are useful in the therapy and/or prevention of illnesses with known over-expression of kinases of the Aurora family preferably Aurora A, especially in the therapy and / or prevention of illnesses mentioned above. The activity of the present compounds as inhibitors of the Aurora kinase family is demonstrated by the following biological assay:

### IC₅₀ determination for inhibitors of Aurora A

### Assay principle

Aurora A is a serine threonine kinase involved in spindle assembly and chromosome segregation.

The assay is a typically ELISA-type assay where substrate (GST-Histone H3) is coupled to the assay-plate and is phosphorylated by the kinase. Phosphorylation is detected by a mouse anti-Phosphopeptid mAb and an HRP-labeled anti-mouse pAb. The assay is validated for IC₅₀ -determination.

Kinase activities were measured by Enzyme-Linked Immunosorbent Assay (ELISA): Maxisorp 384-well plates (Nunc) were coated with recombinant fusion protein comprising residues 1-15 of HistoneH3 fused to the N-terminus of Glutathione-S-Transferase. Plates were then blocked with a solution of 1 mg/mL I-block (Tropix cat# T2015 - highly purified form of casein) in phosphate-buffered saline. Kinase reactions were carried out in the wells of the ELISA plate by combining an appropriate amount of mutant Aurora A kinase with test compound and 30 µM ATP. The reaction buffer was 10X Kinase Buffer (Cell Signaling cat # 9802) supplemented with 1 µg/mL I-block. Reactions were stopped after 40 minutes by addition of 25 mM EDTA. After washing, substrate phosphorylation was detected by addition of anti-phospho-Histone H3 (Ser 10) 6G3 mAb (Cell Signaling cat #9706) and sheep anti-mouse pAb-HRP (Amersham cat# NA931V), followed by colorimetric development with TMB (3,3',5,5'-tetramethylbenzidine from Kirkegaard & Perry Laboratories). After readout of the adsorbance, IC₅₀ values were calculated using a non-linear curve fit (XLfit software (ID Business Solution Ltd., Guilford, Surrey, UK)). The results are shown in Table 1.

**Results: Table 1**

| **Example No.** | **IC50 Aurora A kinase inhibition [µM]** |
|---|---|
| 5 | 0.017 |
| 9 | 0.012 |
| 11 | 0.016 |
| 16 | 0.055 |
| 17 | 0.014 |
| 13, 18, 19, 20, 21, 22, 24, 26, 28 | 0.001-0.500 |

### Antiproliferative activity

The activity of the present compounds as antiproliferative agents is demonstrated by the following biological assay:
CellTiter-Glo^{™} assay in HCT 116 cells
The CellTiter-Glo^{™} Luminescent Cell Viability Assay (Promega) is a homogeneous method of determining the number of viable cells in culture based on quantitation of the ATP present, which signals the presence of metabolically active cells.

HCT 116 cells (human colon carcinoma, ATCC-No. CCl-247) were cultivated in RPMI 1640 medium with GlutaMAX^{™} I (Invitrogen, Cat-No. 61870-010), 2,5 % Fetal Calf Serum (FCS, Sigma Cat-No. F4135 (FBS)); 100Units/ml penicillin/100µg/ml streptomycin (= Pen/Strep from Invitrogen Cat. No. 15140). For the assay the cells were seeded in 384 well plates, 1000 cells per well, in the same medium. The next day the test compounds were added in various concentrations ranging from 30 µM to 0.0015 µM (10 concentrations, 1:3 diluted). After 5 days the CellTiter-Glo^{™} assay was done according to the instructions of the manufacturer (CellTiter-Glo^{™} Luminescent Cell Viability Assay, from Promega). In brief: the cell-plate was equilibrated to room temperature for approximately 30 minutes and than the CellTiter-Glo^{™} reagent was added. The contents were carefully mixed for 15 minutes to induce cell lysis. After 45 minutes the luminescent signal was measured in Victor 2, (scanning multiwell spectrophotometer, Wallac).

### Details:

### 1st. day:

- Medium: RPMI 1640 with GlutaMAX^{™} I (Invitrogen, Cat-Nr. 61870), 5 % FCS (Sigma Cat.-No. F4135), Pen/Strep (Invitrogen, Cat No. 15140).
- HCT116 (ATCC-No. CCl-247): 1000 cells in 60 µl per well of 384 well plate (Greiner 781098, µClear-plate white)
- After seeding incubate plates 24 h at 37°C, 5% CO₂

### 2nd. day: Induction (Treatment with compounds, 10 concentrations):

In order to achieve a final concentration of 30 µM as highest concentration 3,5 µl of 10 mM compound stock solution were added directly to 163 µl media. Then step e) of the dilution procedure described below, was followed.

In order to achieve the second highest to the lowest concentrations, a serial dilution with dilution steps of 1:3 was followed according to the procedure (a -e) as described here below:
a) for the second highest concentration add 10 µl of 10 mM stock solution of compound to 20 µl dimethylsulfoxide (DMSO)
b) dilute 8x 1:3 (always 10 µl to 20 µl DMSO) in this DMSO dilution row (results in 9 wells with concentrations from 3333,3 µM to 0.51 µM)
c) dilute each concentration 1: 47,6 (3,5 µl compound dilution to 163 µl media)
e) add 10 µl of every concentration to 60 µl media in the cell plate resulting in final concentration of DMSO : 0.3 % in every well and resulting in 10 final concentration of compounds ranging from 30 µM to 0.0015 µM.

- Each compound is tested in triplicate.
- Incubate 120 h (5 days) at 37°C, 5% CO₂

### Analysis:

- Add 30 µl CellTiter-Glo^{™} Reagent (prepared from CellTiter-Glo^{™} Buffer and CellTiter-Glo^{™} Substrate (lyophilized) purchased from Promega) per well,
- shake 15 minutes at room temperature
- incubate further 45 minutes at room temperature without shaking

### Measurement:

- Victor 2 scanning multiwell spectrophotometer (Wallac), Luminescence mode (0.5 sec/read, 477 nm)
- Determine IC50 using a non-linear curve fit (XLfit software (ID Business Solution Ltd., Guilford, Surrey, UK))

With all compounds a significant inhibition of HCT 116 cell viability was detected, which is exemplified by the compounds shown in Table 2.

**Results: Table 2**

| **Example No.** | **IC50 HCT 116 [µM]** |
|---|---|
| 2 | 0.430 |
| 4 | 0.224 |
| 6 | 0.682 |
| 9 | 0.108 |
| 15 | 1.504 |
| 24 | 0.741 |
| 28 | 0.338 |
| 1, 3, 7, 8, 11, 13, 16, 17, 18, 21, 23, 25, 26 | 0.100-2.000 |

The compounds according to this invention and their pharmaceutically acceptable salts can be used as medicaments, e.g. in the form of pharmaceutical compositions. The pharmaceutical compositions can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The above-mentioned pharmaceutical compositions can be obtained by processing the compounds according to this invention with pharmaceutically inert, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

A pharmaceutical compositions comprise e.g. the following:

### a) Tablet Formulation (Wet Granulation):

| **Item** | **Ingredients** | **Mg/tablet** | | | |
|---|---|---|---|---|---|
| 1. | Compound of formula I | 5 | 25 | 100 | 500 |
| 2. | Lactose Anhydrous DTG (direct tabletting grade) | 125 | 105 | 30 | 150 |
| 3. | Sta-Rx 1500 (pre-gelatinized starch powder) | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

### Manufacturing Procedure:

1. Mix items 1, 2, 3 and 4 and granulate with purified water.
2. Dry the granules at 50°C.
3. Pass the granules through suitable milling equipment.
4. Add item 5 and mix for three minutes; compress on a suitable press.

### b) Capsule Formulation:

| **Item** | **Ingredients** | **mg/capsule** | | | |
|---|---|---|---|---|---|
| 1. | Compound of formula I | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

### Manufacturing Procedure:

1. Mix items 1, 2 and 3 in a suitable mixer for 30 minutes.
2. Add items 4 and 5 and mix for 3 minutes.
3. Fill into a suitable capsule.

### c) Micro suspension

1. Weigh 4.0 g glass beads in custom made tube GL 25, 4 cm (the beads fill half of the tube).
2. Add 50 mg compound, disperse with spatulum and vortex.
3. Add 2 ml gelatin solution (weight beads: gelatin solution = 2:1) and vortex.
4. Cap and wrap in aluminum foil for light protection.
5. Prepare a counter balance for the mill.
6. Mill for 4 hours, 20/s in a Retsch mill (for some substances up to 24 hours at 30/s).
7. Extract suspension from beads with two layers of filter (100 µm) on a filter holder, coupled to a recipient vial by centrifugation at 400 g for 2 min.
8. Move extract to measuring cylinder.
9. Repeat washing with small volumes(here 1 ml steps) until final volume is reached or extract is clear.
10. Fill up to final volume with gelatin and homogenize.

The following examples and references are provided to aid the understanding of the present invention, the true scope of which is set forth in the appended claims. It is understood that modifications can be made in the procedures set forth without departing from the spirit of the invention.

### Experimental Procedures:

### A: starting materials

### A1 . Preparation of 5,6-diamino-1-ethyl-3,3-dimethyl-1,3-dihydro-indol-2-one

### i) 1-Ethyl-3,3-dimethyl-6-nitro-1,3-dihydro-indol-2-one

A solution of 3,3-dimethyl-6-nitro-1,3-dihydro-indol-2-one (6g, 29.10 mmol) in anhydrous *N*,*N*-dimethylformamide (DMF) (35 ml) was treated with sodium hydride. The resulting suspension was stirred for 1 h at 60°C. A solution of bromo-ethane (2.17 mL, 3.17 g, 29.10 mmol) in DMF (10 ml) was added. The mixture was allowed to cool to room temperature and stirred for 1 h. After removal of the solvent the mixture was quenched with water (100 ml) and extracted with ethyl acetate (3 x 100 ml). The extract was dried over Na₂SO₄, evaporated and the crude product was purified by column chromatography on silica gel. Elution with ethyl acetate/*n*-heptane (1:3) yielded 5.94 g (87%) of a yellow solid.
MS: M = 235.3 (ESI+)
¹H-NMR (400 MHz, DMSO): δ (ppm) = 1.16 (t, 3H), 1.32 (s, 6 H), 3.81 (q, 2H), 7.66 (d, 1H), 7.86 (s, 1H), 7.97 (d, 1H)

### ii) 6-Amino-1-ethyl-3,3-dimethyl-1,3-dihydro-indol-2-one

To a solution of 1-ethyl-3,3-dimethyl-6-nitro-1,3-dihydro-indol-2-one (5.9 g, 25.19 mmol) in methanol/tetrahydrofuran (THF) (1:1, 80 ml) palladium on charcoal (10 %, 1.2 g) was added and the mixture hydrogenated at room temperature for 4 h. After filtration and evaporation of the solvents 5.05 g (98%) 6-amino-1-ethyl-3,3-dimethyl-1,3-dihydro-indol-2-one was isolated as white solid.
MS: M = 205.0 (API+)
¹H-NMR (400 MHz, DMSO): δ (ppm) = 1.11 (t, 3H), 1.17 (s, 6H), 3.58 (q, 2H), 5.12 (br, 2H), 6.21 (d, 1H), 6.25 (s, 1H), 6.92 (d, 1H)

### iii) N-(1-Ethyl-3,3-dimethyl-2-oxo-2,3-dihydro-1H-indol-6-yl)-acetamide

A solution of 6-amino-1-ethyl-3,3-dimethyl-1,3-dihydro-indol-2-one (5.05 g, 24.72 mmol) in acetic anhydride (80 ml) was stirred at room temperature for 4 h. The mixture was poured onto ice water (150 ml), allowed to warm to room temperature and was stirred again for 2 h. After extraction with ethyl acetate (3 x 100 ml), the combined organic layers were washed with sat. NaHCO₃-solution (3 x 100 ml), brine (100 ml) and dried over sodium sulfate. After removal of the solvent the crude product was purified by column chromatography on silica gel (ethyl acetate/*n*-heptane 1:1) yielding 5.6 g (91 %) *N*-(1-ethyl-3,3-dimethyl-2-oxo-2,3-dihydro-1H-indol-6-yl)-acetamide as light yellow solid.
MS: M = 247.1 (API+)
¹H-NMR (400 MHz, DMSO): δ (ppm) = 1.13 (t, 3H), 1.23 (s, 6H), 2.04 (s, 3H), 3.63 (q, 2H), 7.12 (d, 1 H), 7.23 (d, 1H), 7.37 (s, 1H), 9.97 (br, 1H)

### iv) N-(1-ethyl-3,3-dimethyl-5-nitro-2-oxo-2,3-dihydro-1H-indol-6-yl)-acetamide

To a solution of *N*-(1-ethyl-3>3-dimethyl-2-oxo-2,3-dihydro-1H-indol-6-yl)-acetamide ( 5.6 g, 22.73 mmol) in acetic anhydride (70 ml) nitric acid (100 %, 1.96 g, 1.29 ml, 31.2 mmol) was added at 0 °C. The mixture was stirred for 30 min, then poured onto ice water (150 ml). After stirring for 4 h the mixture was extracted with ethyl acetate (3 x 100 ml). The combined organic layers were washed with sodium hydroxide solution (1M, 100 ml) and water (100 ml), dried over sodium sulfate and concentrated. The crude product was purified by column chromatography on silica gel (ethyl acetate/n-heptane 1:1) to yield 5.2 g (78 %) N-(1-ethyl-3,3-dimethyl-5-nitro-2-oxo-2,3-dihydro-1H-indol-6-yl)-acetamide as a yellow solid.
MS: M = 292.0 (API+)
¹H-NMR (400 MHz, DMSO): δ (ppm) = 1.16 (t, 3H), 1.31 (s, 6H), 2.13 (s, 3H), 3.71 (m, 2H), 7.54 (s, 1 H), 8.12 (s, 1H), 10.39 (br, 1H)

### v) 6-Amino-1-ethyl-3,3-dimethyl-5-nitro-1,3-dihydro-indol-2-one

*N*(1-ethyl-3,3-dimethyl-5-nitro-2-oxo-2,3-dihydro-1H-indol-6-yl)-acetamide (5.2 g, 17.85 mmol) was dissolved in ethanol (40 ml). After addition of hydrochloric acid (25 %, 8 ml, 81.44 mmol) the mixture was stirred under reflux for 3 h. The reaction mixture was allowed to cool down to room temperature and then quenched with water (80 ml). The yellow precipitate was isolated by suction and washed with ethanol/water (1:1). The solid was dissolved in ethyl acetate, dried over sodium sulfate and concentrated to yield 4.15 g (93 %) 6-amino-1-ethyl-3,3-dimethyl-5-nitro-1,3-dihydro-indol-2-one as a orange solid.
MS: M = 250.0 (API+)
¹H-NMR (400 MHz, DMSO): δ (ppm) = 1.15 (t, 3H), 1.27 (s, 6H), 3.64 (m, 2H), 6.54 (s, 1H), 7.67 (br, 2H), 7.95 (s, 1H)

### vi) 5,6-Diamino-1-ethyl-3,3-dimethyl-1,3-dihydro-indol-2-one

To a solution of 6-amino-1-ethyl-3,3-dimethyl-5-nitro-1,3-dihydro-indol-2-one (4.15 g, 16.65 mmol) in ethanol (80 ml) PtO₂ (0.4 g) was added and the mixture hydrogenated at room temperature for 3.5 h. After filtration and evaporation of the solvents 3.25 g (89 %) 5,6-diamino-1-ethyl-3,3-dimethyl-1,3-dihydro-indol-2-one was isolated as orange solid.
MS: M = 220.0 (API+)
¹H-NMR (400 MHz, DMSO): δ (ppm) = 1.10 (t, 3H), 1.13 (s, 6H), 3.53 (m, 2H), 4.08 (br, 2H), 4.48 (br, 2H), 6.27 (s, 1H), 6.50 (s, 1H)

### A2. Preparation of 5,6-diamino-1,3,3-trimethyl-1,3-dihydro-indol-2-one

5,6-diamino-1,3,3-trimethyl-1,3-dihydro-indol-2-one was prepared in an analogous 6-step-synthesis as described for 5,6-diamino-1-ethyl-3,3-dimethyl-1,3-dihydro-indol-2-one (A1).
MS: M = 206.1 (API+)
¹H-NMR (400 MHz, DMSO): δ (ppm) = 1.57 (s, 6H), 3.43 (s, 3H), 4.94 (br, 4H), 6.66 (s, 1H), 6.95 (s, 1H)

### A3. Preparation of 5,6-diamino-3,3-dimethyl-1-propyl-1,3-dihydro-indol-2-one

5,6-diamino-3,3-dimethyl-1-propyl-1,3-dihydro-indol-2-one was prepared in an analogous 6-step-synthesis as described for 5,6-diamino-1-ethyl-3,3-dimethyl-1,3-dihydro-indol-2-one (A1).
MS: M = 234.1 (API+)
¹H-NMR (400 MHz, DMSO): δ (ppm) = 0.82 (t, 3H), 1.15 (s, 6H), 1.58 (m, 2H), 3.46 (q, 2H), 4.16 (br, 2H), 4.45 (br, 2H), 6.27 (s, 1H), 6.50 (s, 1H)

### A4. Preparation of 5,6-diamino-1-isopropyl-3,3-dimethyl-1,3-dihydro-indol-2-one

5,6-diamino-3,3-dimethyl-1-isopropyl-1,3-dihydro-indol-2-one was prepared in an analogous 6-step-synthesis as described for 5,6-diamino-1-ethyl-3,3-dimethyl-1,3-dihydro-indol-2-one (A1).
MS: M = 234.1 (API+)
¹H-NMR (400 MHz, DMSO): δ (ppm) = 1.12 (s, 6H), 1.33 (d, 6H), 4.09 (br, 2H), 4.40 (m, 1H), 4.46 (br, 2H), 6.46 (s, 1H), 6.48 (s, 1H)

### A5. Preparation of 5,6-diamino-3,3-dimethyl-1-(3-morpholin-4-yl-propyl)-1,3-dihydro-indol-2-one

5,6-diamino-3,3-dimethyl-1-(3-morpholin-4-yl-propyl)-1,3-dihydro-indol-2-one was prepared in an analogous 6-step-synthesis as described for 5,6-diamino-1-ethyl-3,3-dimethyl-1,3-dihydro-indol-2-one (A1).
MS: M = 319.1 (API+)
¹H-NMR (400 MHz, DMSO): δ (ppm) = 1.14 (s, 6H), 1.70 (m, 2H), 2.26 (t, 2H), 2.33 (m, 4H), 3.56 (m, 6H), 4.39 (br, 4H), 6.28 (s, 1H), 6.50 (s, 1H)

### A6. Preparation of 5,6-diamino-3,3-diethyl-1-isopropyl-1,3-dihydro-indol-2-one

### i) 3,3-Diethyl-5-nitro-1,3-dihydro-indol-2-one

To a solution of 3,3-diethyl-1,3-dihydro-indol-2-one (10.0g, 52.84mmol, Mertens et al., J.Med.Chem. 30 (1987) 1279-1287) in conc. sulfuric acid (50 ml) was added slowly a mixture of nitric acid (65 %, 5.12g, 3.63ml, 52.84mmol) and conc. sulfuric acid (10ml) at 0 °C. After 2h at room temperature the mixture was poured into ice water. The precipitate was filtered off, washed with water and dried to yield 11.7g 3,3-diethyl-5-nitro-1,3-dihydro-indol-2-one (49.95mmol, 94%).
MS: M = 235.1 (ESI+)

### ii) 3,3-Diethyl-1-isopropyl-5-nitro-1,3-dihydro-indol-2-one

A solution of 3,3-diethyl-5-nitro-1,3-dihydro-indol-2-one (11.7g, 49.95mmol) in anhydrous *N*,*N*-dimethylformamide (DMF) (60ml) was treated with sodium hydride (1.558g, 64.93mmol). The resulting suspension was stirred for 1 h at 60°C. A solution of 2-iodo-propane (4.99ml, 8.49g, 49.95mmol) was added. The mixture was kept at 60°C for further 3h, allowed to cool to room temperature poured into ice water. The precipitate was filtered off, washed with water and dried to yield 12.6g 3,3-diethyl-1-isopropyl-5-nitro-1,3-dihydro-indol-2-one (45.60mmol, 91%)
MS: M = 277.1 (ESI+)

### iii) 5-Amino-3,3-diethyl-1-isopropyl-1,3-dihydro-indol-2-one

To a solution of 3,3-diethyl-1-isopropyl-5-nitro-1,3-dihydro-indol-2-one (12.6g, 45.60mmol) in methanol/tetrahydrofuran (THF) (1:1, 80 ml) palladium on charcoal (10 %, 1.2 g) was added and the mixture hydrogenated at room temperature for 4 h. After filtration of the catalyst the solvent was evaporated and the residue triturated with iso-hexane to yield 9.7g 5-amino-3,3-diethyl-1-isopropyl-1,3-dihydro-indol-2-one (39.37mmol, 86%).
MS: M = 247.1 (ESI+)

### iv) N-(3,3-Diethyl-1-isopropyl-2-oxo-2,3-dihydro-1H-indol-5-yl)-acetamide

A solution of 5-amino-3,3-diethyl-1-isopropyl-1,3-dihydro-indol-2-one (9.7g, 39.37mmol) in acetic anhydride (57ml) was stirred at room temperature for 4 h. The mixture was poured into ice water, allowed to warm to room temperature and was stirred again for 2 h. After extraction with ethyl acetate, the combined organic layers were washed with aqueous NaOH solution (1M) and brine and dried over sodium sulfate. After removal of the solvent the crude product was triturated with iso-hexane to yield 10.4g N-(3,3-Diethyl-1-isopropyl-2-oxo-2,3-dihydro-1H-indol-5-yl)-acetamide (36.06mmol, 91%)
MS: M = 289.2 (ESI+)

### v) N-(3,3-Diethyl-1-isopropyl-6-nitro-2-oxo-2,3-dihydro-1H-indol-5-yl)-acetamide

To a solution of N-(3,3-diethyl-1-isopropyl-2-oxo-2,3-dihydro-1H-indol-5-yl)-acetamide (10.4g, 36.06mmol) in conc. sulfuric acid (50ml) was added slowly a mixture of nitric acid (65%, 3.84g, 2.72ml, 39.67mmol) and conc. sulfuric acid (10ml) at 0 °C. After 2h at room temperature the mixture was poured into ice water. The precipitate was filtered off, washed with water and dried. The crude material was purified by silica gel chromatography (isohexane/ ethyl acetate 1:1) to yield 2.2g N-(3,3-diethyl-1-isopropyl-6-nitro-2-oxo-2,3-dihydro-1H-indol-5-yl)-acetamide (6.60mmol, 18%) besides undesired N-(3,3-diethyl-1-isopropyl-7-nitro-2-oxo-2,3-dihydro-1H-indol-5-yl)-acetamide (5.5g).
MS: M = 332.2 (ESI-)

### vi) 5-Amino-3,3-diethyl-1-isopropyl-6-nitro-1,3-dihydro-indol-2-one

N-(3,3-diethyl-1-isopropyl-6-nitro-2-oxo-2,3-dihydro-1H-indol-5-yl)-acetamide (2.2 g, 6.60mmol) was dissolved in ethanol (50 ml). After addition of hydrochloric acid (25%, 3.2ml, 33.0mmol) the mixture was heated under reflux for 3h. Most of the solvent was evaporated and water was added. The mixture was weakly alkalized by addition of aqueous NaOH solution. The mixture was extracted with ethyl acetate, the combined organic phases were dried over magnesium sulfate and the solvent was evaporated to yield 1.9g 5-amino-3,3-diethyl-1-isopropyl-6-nitro-1,3-dihydro-indol-2-one (6.52mmol, 99%).
MS: M = 290.1 (ESI-)

### vii) 5,6-Diamino-3,3-diethyl-1-isopropyl-1,3-dihydro-indol-2-one

To a solution of 5-amino-3,3-diethyl-1-isopropyl-6-nitro-1,3-dihydro-indol-2-one (1.9g, 6.52mmol) in methanol/tetrahydrofuran (THF) (1:1, 80 ml) palladium on charcoal (10 %, 1.2 g) was added and the mixture hydrogenated at room temperature for 4 h. After filtration the solvent was evaporated and the residue triturated with iso-hexane to yield 1.7g 5,6-diamino-3,3-diethyl-1-isopropyl-1,3-dihydro-indol-2-one (6.50mmol, 99%).
MS: M = 262.3 (ESI+)
¹H-NMR (400 MHz, DMSO): δ (ppm) = 0.44 (t, 6H), 1.34 (d, 6H), 1.55 (q, 2H), 1.65 (q, 2H), 4.40 (br, 4H), 4.45 (m, 1H), 6.42 (s, 1H), 6.46 (s, 1H)

### A7. Preparation of 5,6-diamino-1,3,3-triethyl-1,3-dihydro-indol-2-one

5,6-Diamino-1,3,3-triethyl-1,3-dihydro-indol-2-one was prepared in an analogous 7-step-synthesis as described for 5,6-diamino-3,3-diethyl-1-isopropyl-1,3-dihydro-indol-2-one (A6).
MS: M = 248.1 (API+)
¹H-NMR (400 MHz, DMSO): δ (ppm) = 0.43 (t, 6H), 1.08 (t, 3H), 1.55 (q, 2H), 1.63 (q, 2H), 3.54 (q, 2H), 4.10 (br, 2H), 4.48 (br, 2H), 6.27 (s, 1H), 6.43 (s, 1H)

### A8. Preparation of 5-Benzyl-4,5,6,7-tetrahydro-1H-pyrazolo[4,3-c]pyridine-3-carboxylic acid ethyl ester

### i) (1-Benzyl-4-oxo-piperidin-3-yl)-oxo-acetic acid ethyl ester

Sodium (1.340g, 58.28mmol) was added to ice-cooled ethanol (50ml) under a nitrogen atmosphere. After 15h at 0°C, the solution was cooled to -10°C and oxalic acid diethyl ester (7.722g, 52.84mmol) was added dropwise. Then a solution of 1-benzyl-piperidin-4-one (9.995g, 52.81mmol) in ethanol (30ml) was added within an hour. The reaction mixture was warmed to room temperature and after 5h the solvent was evaporated to give crude (1-benzyl-4-oxo-piperidin-3-yl)-oxo-acetic acid ethyl ester (17.07g) which was used for the next reaction without further purification.
MS: M = 290.1 (ESI+)

### ii) 5-Benzyl-4,5,6,7-tetrahydro-1H-pyrazolo[4,3-c]pyridine-3-carboxylic acid ethyl ester

To a solution of (1-benzyl-4-oxo-piperidin-3-yl)-oxo-acetic acid ethyl ester (17.07g) in acetic acid (60ml) at 0°C was added hydrazine hydrate (2.959g, 59.10mmol). After heating to 120°C under reflux for 6h the reaction mixture was cooled to room temperature and treated with water (150ml) and ethyl acetate (150ml). The organic phase was washed with saturated aqueous bicarbonate solution until pH 7-8. The combined aqueous phases were reextracted twice with ethyl acetate and the combined organic phases were dried over MgSO4. The solvent was evaporated and the residue subjected to silica gel chromatography (0.5% triethylamine in ethyl acetate) to yield 3.00g 5-benzyl-4,5,6,7-tetrahydro-1H-pyrazolo[4,3-c]pyridine-3-carboxylic acid ethyl ester (10.51mmol), 17.8%)
MS: M = 286.1 (API+)

### A9. Preparation of 5-Benzyl-1,4,5,6-tetrahydro-pyrrolo[3,4-c]pyrazole-3-carboxylic acid ethyl ester

### i) (1-Benzyl-4-oxo-pyrrolidin-3-yl)-oxo-acetic acid ethyl ester

In an analogous manner as described for A8 i) (1-benzyl-4-oxo-pyrrolidin-3-yl)-oxo-acetic acid ethyl ester was prepared from 1-benzyl-3-pyrrolidinone.
MS: M = 276.1 (API+)

### ii) 5-Benzyl-1,4,5,6-tetrahydro-pyrrolo[3,4-c]pyrazole-3-carboxylic acid ethyl ester

In an analogous manner as described for A8 ii) 5-benzyl-1,4,5,6-tetrahydro-pyrrolo[3,4-c]pyrazole-3-carboxylic acid ethyl ester was prepared from (1-benzyl-4-oxo-pyrrolidin-3-yl)-oxo-acetic acid ethyl ester.
MS: M = 272.0 (API+)

### A10. Preparation of 1,4,5,6-Tetrahydro-pyrrolo[3,4-c]pyrazole-3-carboxylic acid ethyl ester

To a solution of 5-benzyl-1,4,5,6-tetrahydro-pyrrolo[3,4-c]pyrazole-3-carboxylic acid ethyl ester (A9, 2.74g, 10.099mmol) in ethanol/ tetrahydrofuran (THF) (1:2, 60ml) palladium on charcoal (10 %, 0.55g) was added and the mixture hydrogenated at room temperature and 44mbar for 4.5h. After filtration of the catalyst the solvent was evaporated and the residue was recrystallized from diethyl ether to yield 1.50g 1,4,5,6-tetrahydro-pyrrolo[3,4-c]pyrazole-3-carboxylic acid ethyl ester (8.278mmol, 82%).
MS: M = 182.2 (ESI+)

### A11. Preparation of 5-(2,6-Diethyl-phenylcarbamoyl)-1,4,5,6-tetrahydro-pyrrolo[3,4-c]pyrazole-3-carboxylic acid

### i) 5-(2,6-Diethyl-phenylcarbamoyl)-1,4,5,6-tetrahydro-pyrrolo[3,4-c]pyrazole-3-carboxylic acid ethyl ester

To a solution of 1,4,5,6-tetrahydro-pyrrolo[3,4-c]pyrazole-3-carboxylic acid ethyl ester (A10, 200mg, 1.104mmol) in DMF (3ml) was added triethylamine (223.4mg, 308µl, 2.208mmol) and a solution of 1,3-diethyl-2-isocyanato-benzene (193.4mg, 191µl, 1.104mmol) in DMF (1ml). After 1h at room temperature the mixture was treated with water (40ml). The aqueous phase was extracted with ethyl acetate, the combined organic phases were dried over MgSO₄ and the solvent was evaporated. The residue was purified by silica gel chromatography (ethyl acetate/ heptane 2:1) to yield 304mg 5-(2,6-diethyl-phenylcarbamoyl)-1,4,5,6-tetrahydro-pyrrolo[3,4-c]pyrazole-3-carboxylic acid ethyl ester (0.853mmol, 77%).
MS: M = 357.2 (ESI+)

### ii) 5-(2,6-Diethyl-phenylcarbamoyl)-1,4,5,6-tetrahydro-pyrrolo[3,4-c]pyrazole-3-carboxylic acid

To a solution of 5-(2,6-diethyl-phenylcarbamoyl)-1,4,5,6-tetrahydro-pyrrolo[3,4-c]pyrazole-3-carboxylic acid ethyl ester (245mg, 0.687mmol) in THF (4ml) was added NaOH (2N, 2ml, 4.0mmol) and heated under reflux for 2h. The mixture was cooled to room temperature and acidified with HCl (2N). The aqueous phase was extracted ethyl acetate, the combined organic phases were washed with brine, dried over MgSO₄ and the solvent was evaporated to yield 225mg 5-(2,6-diethyl-phenylcarbamoyl)-1,4,5,6-tetrahydro-pyrrolo[3,4-c]pyrazole-3-carboxylic acid (0.685mmol, 99%).
MS: M = 329.1 (ESI+)

### A12. Preparation of 5-Cyclopropanecarbonyl-1,4,5,6-tetrahydro-pyrrolo[3,4-c]pyrazole-3-carboxylic acid

### i) 5-Cyclopropanecarbonyl-1,4,5,6-tetrahydro-pyrrolo[3,4-c]pyrazole-3-carboxylic acid ethyl ester

To a solution of 1,4,5,6-tetrahydro-pyrrolo[3,4-c]pyrazole-3-carboxylic acid ethyl ester (A10, 100mg, 0.552mmol) in THF (2ml) were added cyclopropanecarbonyl chloride (63.5mg, 0.607mmol) and diisopropylethylamine (178.3mg, 240µl, 1.380mmol) at 0°C. After stirring at room temperature for 12h the mixture was quenched with NaOH (2N, 0.5ml) and water (5ml). The aqueous phase was extracted ethyl acetate, the combined organic phases were dried over Na₂SO₄ and the solvent was evaporated to yield 98mg 5-cyclopropanecarbonyl-1,4,5,6-tetrahydro-pyrrolo[3,4-c]pyrazole-3-carboxylic acid ethyl ester (0.393mmol, 71%)
MS: M = 250.0 (API+)

### ii) 5-Cyclopropanecarbonyl-1,4,5,6-tetrahydro-pyrrolo[3,4-c]pyrazole-3-carboxylic acid

In an analogous manner as described for A11 ii) 5-cyclopropanecarbonyl-1,4,5,6-tetrahydro-pyrrolo[3,4-c]pyrazole-3-carboxylic acid was prepared from 5-cyclopropanecarbonyl-1,4,5,6-tetrahydro-pyrrolo[3,4-c]pyrazole-3-carboxylic acid ethyl ester.

### A13. 5-(2-Thiophen-2-yl-acetyl)-1,4,5,6-tetrahydro-pyrrolo[3,4-c]pyrazole-3-carboxylic acid

### i) 5-(2-Thiophen-2-yl-acetyl)-1,4,5,6-tetrahydro-pyrrolo[3,4-c]pyrazole-3-carboxylic acid ethyl ester

In an analogous manner as described for A12 i) 5-(2-thiophen-2-yl-acetyl)-1,4,5,6-tetrahydro-pyrrolo[3,4-c]pyrazole-3-carboxylic acid ethyl ester was prepared from 1,4,5,6-tetrahydro-pyrrolo[3,4-c]pyrazole-3-carboxylic acid ethyl ester (A10) and thiophen-2-yl-acetyl chloride.
MS: M = 306.2 (API+)

### ii) 5-(2-Thiophen-2-yl-acetyl)-1,4,5,6-tetrahydro-pyrrolo[3,4-c]pyrazole-3-carboxylic acid

In an analogous manner as described for A11 ii) 5-(2-thiophen-2-yl-acetyl)-1,4,5,6-tetrahydro-pyrrolo[3,4-c]pyrazole-3-carboxylic acid was prepared from 5-(2-thiophen-2-yl-acetyl)-1,4,5,6-tetrahydro-pyrrolo[3,4-c]pyrazole-3-carboxylic acid ethyl ester.
MS: M = 278.1 (ESI+)

### A14. 5-(4-Fluoro-benzenesulfonyl)-1,4,5 6-tetrahydro-pyrrolo[3,4-c]pyrazole-3-carboxylic acid

### i) 5-(4-Fluoro-benzenesulfonyl)-1,4,5,6-tetrahydro-pyrrolo[3,4-c]pyrazole-3-carboxylic acid ethyl ester

In an analogous manner as described for A12 i) 5-(4-Fluoro-benzenesulfonyl)-1,4,5,6-tetrahydro-pyrrolo[3,4-c]pyrazole-3-carboxylic acid ethyl ester was prepared from 1,4,5,6-tetrahydro-pyrrolo[3,4-c]pyrazole-3-carboxylic acid ethyl ester (A10) and 4-fluoro-benzenesulfonyl chloride.
MS: M = 340.0 (ESI+)

### ii) 5-(4-Fluoro-benzenesulfonyl)-1,4,5,6-tetrahydro-pyrrolo[3,4-c]pyrazole-3-carboxylic acid

In an analogous manner as described for A11 ii) 5-(4-fluoro-benzenesulfonyl)-1,4,5,6-tetrahydro-pyrrolo[3,4-c]pyrazole-3-carboxylic acid was prepared from 5-(4-fluoro-benzenesulfonyl)-1,4,5,6-tetrahydro-pyrrolo [3,4-c]pyrazole-3-carboxylic acid ethyl ester.
MS: M = 310.0 (ESI-)

### A15.1,4,6,7-Tetrahydro-thiopyrano[4,3-c]pyrazole-3-carboxylic acid

### i) Oxo-(4-oxo-tetrahydro-thiopyran-3-yl)-acetic acid ethyl ester

In an analogous manner as described for A8 i) oxo-(4-oxo-tetrahydro-thiopyran-3-yl)-acetic acid ethyl ester was prepared from tetrahydro-thiopyran-4-one.
MS: M = 215.0 (API-)

### ii) 1,4,6,7-Tetrahydro-thiopyrano[4,3-c]pyrazole-3-carboxylic acid ethyl ester

In an analogous manner as described for A8 ii) 1,4,6,7-tetrahydro-thiopyrano[4,3-c]pyrazole-3-carboxylic acid ethyl ester was prepared from oxo-(4-oxo-tetrahydrothiopyran-3-yl)-acetic acid ethyl ester.
MS: M = 213.1 (ESI+)

### iii) 1,4,6,7-Tetrahydro-thiopyrano[4,3-c]pyrazole-3-carboxylic acid

In an analogous manner as described for A11 ii) 1,4,6,7-tetrahydro-thiopyrano[4,3-c]pyrazole-3-carboxylic acid was prepared from 1,4,6,7-tetrahydro-thiopyrano[4,3-c]pyrazole-3-carboxylic acid ethyl ester.
MS: M =183.1 (ESI-)

### A15.4,6-Dihydro-1H-thieno[3,4-c]pyrazole-3-carboxylic acid

### i) Oxo-(4-oxo-tetrahydro-thiophen-3-yl)-acetic acid ethyl ester

In an analogous manner as described for A8 i) oxo-(4-oxo-tetrahydro-thiophen-3-yl)-acetic acid ethyl ester was prepared from dihydro-thiophen-3-one.

### ii) 4,6-Dihydro-1H-thieno[3,4-c]pyrazole-3-carboxylic acid ethyl ester

In an analogous manner as described for A8 ii) 4,6-dihydro-1H-thieno[3,4-c]pyrazole-3-carboxylic acid ethyl ester was prepared from oxo-(4-oxo-tetrahydro-thiophen-3-yl)-acetic acid ethyl ester.
MS: M = 199.0 (API+)

### iii) 4,6-Dihydro-1H-thieno[3,4-c]pyrazole-3-carboxylic acid

In an analogous manner as described for A11 ii) 4,6-Dihydro-1H-thieno[3,4-c]pyrazole-3-carboxylic acid was prepared from 4,6-dihydro-1H-thieno[3,4-c]pyrazole-3-carboxylic acid ethyl ester.
MS: M = 171.0 (API+)

### Final products

### Example 1

### 7,7-Dimethyl-2-(1,4,5,6-tetrahydro-cyclopentapyrazol-3-yl)-5,7-dihydro-3H-imidazo [4,5-f] indol-6-one

5,6-Diamino-3,3-dimethyl-1,3-dihydro-indol-2-one (143 mg, 0.75 mmol), and 1,4,5,6-Tetrahydro-cyclopentapyrazole-3-carboxylic acid (114 mg, 0.75 mmol) were mixed with polyphosphoric acid (5.10 g, 53.12 mmol) and phosphorus pentoxide (190 mg, 1.34 mmol) and stirred under nitrogen at 150 °C for 6 h. The mixture was quenched with ice water (25 ml) and the resulting solution was adjusted to pH 7 - 8 by adding aqueous ammonia and then extracted twice with ethyl acetate (3 x 50 ml). The combined organic layers were washed with water (50 ml), dried over sodium sulfate and concentrated. The crude product was purified by HPL chromatography. Yield 37 mg (16%) of a light brown solid.
MS: M = 308.1 (API+)
¹H-NMR (400 MHz, D₆-DMSO): δ (ppm) = 1.29 (s, 6H), 2.52 (m, 2H), 2.71 (m, 2H), 2.81 (m, 2H), 6.88 (br, 1H), 6.95 (br, 1H), 10.23 (br, 1H)

### Example 2

### 5,7,7-Trimethyl-2- 1,4,5,6-tetrahydro-cyclopentapyrazol-3-yl)-5,7-dihydro-3H-imidazo [4,5-f]indol-6-one

In an analogous manner as described for example 1 5,7,7-Trimethyl-2-(1,4,5,6-tetrahydro-cyclopentapyrazol-3-yl)-5,7-dihydro-3H-imidazo [4,5-f] indol-6-one was prepared from the appropriate starting material.
MS: M = 322.0 (API+)
¹H-NMR (400 MHz, D₆-DMSO): δ (ppm) = 1.31 (s, 6H), 2.52 (m, 2H), 2.71 (m, 2H), 2.81 (m, 2H), 3.19 (s, 3H), 6.95 and 7.23 (s, 1H, two tautomeric forms), 7.39 and 7.61 (s, 1H, two tautomeric forms), 12.48 (br, 1H), 12.70 (br, 1H)

### Example 3

### 5-Ethyl-7,7-dimethyl-2-(1,4,5,6-tetrahydro-cyclopentapyrazol-3-yl)-5,7-dihydro-3H-imidazo[4,5-f]indol-6-one

In an analogous manner as described for example 1 5-Ethyl-7,7-dimethyl-2-(1,4,5,6-tetrahydro-cyclopentapyrazol-3-yl)-5,7-dihydro-3H-imidazo [4,5-f]indol-6-one was prepared from the appropriate starting material.
MS: M = 336.2 (API+)
¹H-NMR (400 MHz, D₆-DMSO): δ (ppm) = 1.18 (t, 3H), 1.31 (s, 6H), 2.55 (m, 2H), 2.70 (m, 2H), 2.81 (m, 2H), 3.76 (q, 2H), 6.98 and 7.27 (s, 1H, two tautomeric forms), 7.37 and 7.61 (s, 1H, two tautomeric forms), 12.50 (br, 1H), 12.75 (br, 1H)

### Example 4

### 7,7-Dimethyl-5-propyl-2-(1,4,5,6-tetrahydro-cyclopentapyrazol-3-yl)-5,7-dihydro-3H-imidazo[4,5-f]indol-6-one

In an analogous manner as described for example 1 7,7-Dimethyl-5-propyl-2-(1,4,5,6-tetrahydro-cyclopentapyrazol-3-yl)-5,7-dihydro-3H-imidazo[4,5-f]indol-6-one was prepared from the appropriate starting material.
MS: M = 350.1 (API+)
¹H-NMR (400 MHz,D₆-DMSO : δ (ppm) = 0.85 (m, 3H), 1.31 (s, 6H), 1.66 (m, 2H), 2.52 (m, 2H), 2.71 (m, 2H), 2.81 (m, 2H), 3.69 (t, 2H), 6.98 and 7.27 (s, 1H, two tautomeric forms), 7.37 and 7.61 (s, 1H, two tautomeric forms), 12.41 an 12.50 (br, 1H, two tautomeric forms), 12.70 (br, 1H)

### Example 5

### 5-Isopropyl-7,7-dimethyl-2-(1,4,5,6-tetrahydro-cyclopentapyrazol-3-yl)-5,7-dihydro-3H-imidazo[4,5-f]indol-6-one

In an analogous manner as described for example 1 5-Isopropyl-7,7-dimethyl-2-(1,4,5,6-tetrahydro-cyclopentapyrazol-3-yl)-5,7-dihydro-3H-imidazo[4,5-f]indol-6-one was prepared from the appropriate starting material.
MS: M = 350.1 (API+)
¹H-NMR (400 MHz, D₆-DMSO): δ (ppm) = 1.29 (s, 6H), 1.44 (d, 6 H), 2.51 (m, 2H), 2.71 (m, 2H), 2.81 (m, 2H), 4.57 (m, 1H), 7.1-7.6 (br, tautomeric forms, 2H), 12.75 (br, 2H)

### Example 6

### 7,7-Dimethyl-5-(3-morpholin-4-yl-propyl)-2-(1,4,5,6-tetrahydro-cyclopentapyrazol-3-yl)-5,7-dihydro-3H-imidazo[4,5-f] indol-6-one

In an analogous manner as described for example 1 7,7-Dimethyl-5-(3-morpholin-4-yl-propyl)-2-(1,4,5,6-tetrahydro-cyclopentapyrazol-3-yl)-5,7-dihydro-3H-imidazo[4,5-f]indol-6-one was prepared from the appropriate starting material.
MS: M = 435.2 (API+)
¹H-NMR (400 MHz, D₆-DMSO): δ (ppm) = 1.31 (s, 6H), 1.78 (m, 2H), 2.29 (m, 6H), 2.52 (m, 2H), 2.70 (m, 2H), 2.81 (m, 2H), 3.58 (m, 4H), 3.75 (t, 2H), 6.02 and 7.29 (s, 1H, two tautomeric forms), 7.37 and 7.58 (s, 1H, two tautomeric forms), 12.41 and 12.50 (br, 2H)

### Example 7

### 7,7-Dimethyl-2-(4,5,6,7-tetrahydro-1H-indazol-3-yl)-5,7-dihydro-3H-imidazo[4,5-f]indol-6-one

In an analogous manner as described for example 1 7,7-Dimethyl-2-(4,5,6,7-tetrahydro-1H-indazol-3-yl)-5,7-dihydro-3H-imidazo[4,5-f]indol-6-one was prepared from the appropriate starting material.
MS: M = 322.0 (API+)
¹H-NMR (400 MHz, D₆-DMSO : δ (ppm) = 1.29 (s, 6H), 1.76 (m, 4H), 2.63 (m, 2H), 2.81 (m, 2H), 6.88 and 6.99 (s, 1H, two tautomeric forms), 7.28 and 7.52 (s, 1H, two tautomeric forms), 10.18 and 10.24 (br, 1 H, two tautomeric forms), 12.32 and 12.46 (br, 1H), 12.72 and 12.74 (br, 1H, two tautomeric forms)

### Example 8

### 5-Ethyl-7,7-dimethyl-2-(4,5,6,7-tetrahydro-1H-indazol-3-yl)-5,7-dihydro-3H-imidazo[4,5-f]indol-6-one

In an analogous manner as described for example 1 5-Ethyl-7,7-dimethyl-2-(4,5,6,7-tetrahydro-1H-indazol-3-yl)-5,7-dihydro-3H-imidazo [4,5-f]indol-6-one was prepared from the appropriate starting material.
MS: M = 350.1 (API+)
¹H-NMR (400 MHz, D₆-DMSO): δ (ppm) = 1.19 (t, 3H), 1.30 (s, 6H), 1.76 (m, 4H), 2.63 (m, 2H), 2.82 (m, 2H), 3.75 (q, 2H), 6.95 and 7.29 (s, 1H, two tautomeric forms), 7.35 and 7.63 (s, 1 H, two tautomeric forms), 12.48 and 12.53 (br, 1H), 12.75 (br, 1H)

### Example 9

### 5-Isopropyl-7,7-dimethyl-2-(4,5,6,7-tetrahydro-1H-indazol-3-yl)-5,7-dihydro-3H-imidazo [4,5-f]indol-6-one

In an analogous manner as described for example 1 5-Isopropyl-7,7-dimethyl-2-(4,5,6,7-tetrahydro-1H-indazol-3-yl)-5,7-dihydro-3H-imidazo[4,5-f]indol-6-one was prepared from the appropriate starting material.
MS: M = 364.1 (API+)
¹H-NMR (400 MHz, D₆-DMSO): δ (ppm) = 1.28 (s, 6H), 1.45 (d, 6H), 1.76 (m, 4H), 2.64 (m, 2H), 2.82 (m, 2H), 4.55 (m, 1H), 7.08 and 7.33 (s, 1H, two tautomeric forms), 7.35 and 7.60 (s, 1 H, two tautomeric forms), 12.40 and 12.52 (br, 1H), 12.75 (br, 1H)

### Example 10

### 7,7-Dimethyl-5-(3-morpholin-4-yl-propyl)-2-(4,5,6,7-tetrahydro-1H-indazol-3-yl)-5,7-dihydro-3H-imidazo[4,5-f]indol-6-one

In an analogous manner as described for example 1 7,7-Dimethyl-5-(3-morpholin-4-yl-propyl)-2-(4,5,6,7-tetrahydro-1H-indazol-3-yl)-5,7-dihydro-3H-imidazo[4,5-f]indol-6-one was prepared from the appropriate starting material.
MS: M = 449.1 (API+)
¹H-NMR (400 MHz, D₆-DMSO): δ (ppm) = 1.30 (s, 6H), 1.78 (m, 6H), 2.29 (m, 6H), 2.64 (m, 2H), 2.81 (m, 2H), 3.58 (m, 4H), 3.74 (t, 2H), 7.00 and 7.31 (s, 1H, two tautomeric forms), 7.34 and 7.59 (s, 1 H, two tautomeric forms), 12.51 and 12.53 (br, 1H, two tautomeric forms), 12.74 and 12.76 (br, 1H, two tautomeric forms)

### Example 11

### 5-Ethyl-7,7-dimethyl-2-(1,4,6,7-tetrahydro-pyrano[4,3-c]pyrazol-3-yl)-5,7-dihydro-3H-imidazo[4,5-f]indol-6-one

In an analogous manner as described for example 1 7,7-Dimethyl-5-(3-morpholin-4-yl-propyl)-2-(4,5,6,7-tetrahydro-1H-indazol-3-yl)-5,7-dihydro-3H-imidazo[4,5-f]indol-6-one was prepared from the appropriate starting material.
MS: M = 352.1 (API+)
¹H-NMR (400 MHz, DMSO): δ (ppm) = 1.60 (t, 3H), 1.72 (s, 6H), 3.19 (t, 2H), 4.17 (q, 2H), 4.30 (t, 2H), 5.31 (s, 2H), 7.39 and 7.71 (s, 1H, two tautomeric forms), 7.79 and 8.05 (s, 1H, two tautomeric forms), 13.08 and 13.13 (br, 1H, two tautomeric forms), 13.43 and 13.45 (br, 1H, two tautomeric forms)

### Example 12

### 5,7,7-Triethyl-2-(4,5,6,7-tetrahydro-1H-indazol-3-yl)-5,7-dihydro-3H-imidazo[4,5-f]indol-6-one

In an analogous manner as described for example 1 5,7,7-triethyl-2-(4,5,6,7-tetrahydro-1H-indazol-3-yl)-5,7-dihydro-3H-imidazo[4,5-f]indol-6-one was prepared from the appropriate starting material.
MS: M = 378.3 (ESI+)
¹H-NMR (400 MHz, D₆-DMSO). δ (ppm) = 0.45 (t, 6H), 1.16 (t, 3H), 1.81 (m, 8H), 2.65 (t, 2H), 2.83 (t, 2H), 3.77 (q, 2H), 6.95 and 7.25 (s, 1H, two tautomeric forms), 7.27 and 7.50 (s, 1H, two tautomeric forms), 12.50 and 12.53 (br, 1H, two tautomeric forms, 12.75 and 12.78 (br, 1H, two tautomeric forms)

### Example 13

### 2-(5-Benzyl-4,5,6,7-tetrahydro-1H-pyrazolo [4,3-c]pyridin-3-yl)-5-ethyl-7,7-dimethyl-5,7-dihydro-3H-imidazo [4,5-f] indol-6-one

5,6-Diamino-1-ethyl-3,3-dimethyl-1,3-dihydro-indol-2-one (A1, 1.109g, 5.057mmol), and 5-benzyl-4,5,6,7-tetrahydro-1H-pyrazolo[4,3-c]pyridine-3-carboxylic acid ethyl ester (A8, 1.44g, 5.046mmol) were mixed with polyphosphoric acid (16.82g, 117.6mmol) and phosphorous pentoxide (4.305g, 30.33mmol) and stirred under nitrogen at 150 °C for 12h. The mixture was quenched with ice water (70ml) and the resulting solution was adjusted to pH 7-8 by adding aqueous ammonia. After 3h in the refrigerator the precipitate was filtered off and purified by HPL chromatography to yield 580mg 2-(5-benzyl-4,5,6,7-tetrahydro-1H-pyrazolo[4,3-c]pyridin-3-yl)-5-ethyl-7,7-dimethyl-5,7-dihydro-3H-imidazo[4,5-f]indol-6-one (1.316mmol), 26%)
MS: M = 441.1 (API+)
¹H-NMR (400 MHz, DMSO): δ (ppm) = 1.13 (m, 3H), 1.28 (s, 6H), 2.76 (m, 4H), 3.70 - 3.84 (m, 6H), 6.95 and 7.29 (s, 1H, two tautomeric forms), 7.27 (m, 1H), 7.31 - 7.40 (m, 4H), 7.41 and 7.60 (s, 1H, two tautomeric forms), 12.55 and 12.61 (s, 1H, two tautomeric forms)

### Example 14

### 5-Ethyl-7,7-dimethyl-2-(4,5,6,7-tetrahydro-1H-pyrazolo[4,3-c]pyridin-3-yl)-5,7-dihydro-3H-imidazo[4,5-f]indol-6-one

To a solution of 2-(5-benzyl-4,5,6,7-tetrahydro-1H-pyrazolo[4,3-c]pyridin-3-yl)-5-ethyl-7,7-dimethyl-5,7-dihydro-3H-imidazo[4,5-f]indol-6-one (example 13, 76mg, 0.172mmol) in methanol/ tetrahydrofuran (THF) (1:3, 12ml) palladium on charcoal (10 %, 30mg) was added and the mixture hydrogenated at room temperature and 32mbar for 4 h. After filtration of the catalyst the solvent was evaporated to yield 39.3mg 5-ethyl-7,7-dimethyl-2-(4,5,6,7-tetrahydro-1H-pyrazolo [4,3-c] pyridin-3-yl)-5,7-dihydro-3H-imidazo [4,5-f] indol-6-one (0.112mmol,65%)
MS: M = 351.1 (API+)
¹H-NMR (400 MHz, DMSO): δ (ppm) = 1.18 (t, 3H), 1.30 (s, 6H), 2.69(t, 2H), 3.04 (t, 2H), 3.69 - 3.81 (m, 2H), 4.08 (s, 2H), 6.96 and 7.28 (s, 1H, two tautomeric forms), 7.37 and 7.61 (s, 1H, two tautomeric forms), 12.51 - 12.98 (s, 2H)

### Example 15

### 5-Ethyl-2-{5-[2-(4-fluoro-phenyl)-acetyl]-4,5,6,7-tetrahydro-1H-pyrazolo[4,3-c]pyridin-3-yl}-7,7-dimethyl-5,7-dihydro-3H-imidazo[4,5-f]indol-6-one

To a solution of (4-fluoro-phenyl)-acetic acid (24mg, 0.156mmol) in absolute DMF (1ml) under a nitrogen atmosphere were added N'-(3-dimethylaminopropyl)-N-ethylcarbodiimide hydrochloride (33mg, 0.172mmol) and hydroxybenzotriazole hydrate (26mg, 0.170mmol). After 30 minutes at room temperature 5-ethyl-7,7-dimethyl-2-(4,5,6,7-tetrahydro-1H-pyrazolo[4,3-c]pyridin-3-yl)-5,7-dihydro-3H-imidazo[4,5-f]indol-6-one (example 14, 50mg, 0.143mmol) was added and stirring continued for 2h. The reaction mixture was treated with water and the aqueous phase extracted twice with ethyl acetate. The combined organic phases were washed with bicarbonate solution, dried over MgSO₄ and the solvent was evaporated. The residue was purified by HPL chromatography to yield 31.2mg 5-ethyl-2-{5-[2-(4-fluoro-phenyl)-acetyl]-4,5,6,7-tetrahydro-1H-pyrazolo[4,3-c]pyridin-3-yl}-7,7-dimethyl-5,7-dihydro-3H-imidazo[4,5-f]indol-6-one (0.057mmol, 40%).
MS: M = 487.0 (API+)
¹H-NMR (400 MHz, DMSO): δ (ppm) = 1.18 (t, 3H), 1.30 (s, 6H), 2.62 - 2.77 (d, 2H), 3.67 - 3.93 (m, 6H), 4.76 - 4.95 (d, 2H), 6.97 and 7.31 (s, 1H, two tautomeric forms), 7.04 - 7.20 (m, 2H), 7.22 - 7.35 (m, 2H), 7.37 and 7.69 (s, 1H, two tautomeric forms), 12.56 - 12.85 (s, 1H), 12.88 - 13.19 (s, 1H)

### Example 16

### 5-Ethyl-2-[5-(4-fluoro-benzoyl)-4,5,6,7-tetrahydro-1H-pyrazolo[4,3-c]pyridin-3-yl] -7,7-dimethyl-5,7-dihydro-3H-imidazo [4,5-f]indol-6-one

In an analogous manner as described for example 15 5-ethyl-2-[5-(4-fluorobenzoyl)-4,5,6,7-tetrahydro-1H-pyrazolo[4,3-c]pyridin-3-yl]-7,7-dimethyl-5,7-dihydro-3H-imidazo[4,5-f]indol-6-one was prepared from 5-ethyl-7,7-dimethyl-2-(4,5,6,7-tetrahydro-1H-pyrazolo[4,3-c]pyridin-3-yl)-5,7-dihydro-3H-imidazo[4,5-f]indol-6-one (example 14) and 4-fluoro-benzoic acid.
MS: M = 471.2 (API-)
¹H-NMR (400 MHz, DMSO): δ (ppm) = 1.18 (m, 3H), 1.30 (s, 6H), 2.85 (m, 2H), 3.51 (m, 2H), 3.75 (m, 2H), 4.93 (m, 2H), 6.96 and 7.37 (s, 1H, two tautomeric forms), 7.31 (t, 2H), 7.41 and 7.73 (s, 1H, two tautomeric forms), 7.57 (m, 2H), 12.53 - 12.86 (s, 1H), 13.07 and 13.11 (s, 1H, two tautomeric forms)

### Example 17

### 5-Ethyl-2-[5-(4-fluoro-benzenesulfonyl)-4,5,6,7-tetrahydro-1H-pyrazolo[4,3-c]pyridin-3-yl]-7,7-dimethyl-5,7-dihydro-3H-imidazo[4,5-f]indol-6-one

To a solution of 5-ethyl-7,7-dimethyl-2-(4,5,6,7-tetrahydro-1H-pyrazolo[4,3-c]pyridin-3-yl)-5,7-dihydro-3H-imidazo[4,5-f]indol-6-one (example 14, 60mg, 0.171mmol) in absolute THF (1ml) at 0°C were added 4-fluoro-benzenesulfonyl chloride (37mg, 0.188mmol) and diisopropylethylamine (55.1mg, 0.426mmol) under a nitrogen atmosphere. After 3h at room temperature the solvent was evaporated and the residue purified by HPL chromatography to yield 34mg 5-ethyl-2-[5-(4-fluoro-benzenesulfonyl)-4,5,6,7-tetrahydro-1H-pyrazolo[4,3-c]pyridin-3-yl]-7,7-dimethyl-5,7-dihydro-3H-imidazo[4,5-f]indol-6-one (0.0598mmol, 35%)
MS: M = 508.2 (API-)
¹H-NMR (400 MHz, DMSO): δ (ppm) = 1.19 (t, 3H), 1.31 (s, 6H), 2.78 (t, 2H), 3.50 (t, 2H), 3.76 (t, 2H), 4.51 (s, 2H), 6.97 and 7.36 (s, 1H, two tautomeric forms), 7.47 (t, 2H), 7.38 and 7.70 (s, 1H, two tautomeric forms), 7.90 (t, 2H), 12.61 - 12.88 (s, 1H), 12.89 - 13.19 (s, 1H)

### Example 18

### 5-Ethyl-7,7-dimethyl-2-[5-(morpholine-4-carbonyl)-4,5,6,7-tetrahydro-1H-pyrazolo[4,3-c]pyridin-3-yl]-5,7-dihydro-3H-imidazo[4,5-f]indol-6-one

In an analogous manner as described for example 17 5-ethyl-7,7-dimethyl-2-[5-(morpholine-4-carbonyl)-4,5,6,7-tetrahydro-1H-pyrazolo[4,3-c]pyridin-3-yl]-5,7-dihydro-3H-imidazo[4,5-f]indol-6-one was prepared from 5-ethyl-7,7-dimethyl-2-(4,5,6,7-tetrahydro-1H-pyrazolo[4,3-c]pyridin-3-yl)-5,7-dihydro-3H-imidazo[4,5-f]indol-6-one (example 14) and morpholine-4-carbonyl chloride.
MS: M = 462.2 (API-)
¹H-NMR (400 MHz, DMSO): δ (ppm) = 1.19 (t, 3H), 1.31 (s, 6H), 2.82 (m, 2H), 3.21 (m, 4H), 3.50 (m, 2H), 3.61 (m, 4H), 3.76 (t, 2H), 4.55 (s, 2H), 6.96 and 7.33 (s, 1H, two tautomeric forms), 7.37 and 7.66 (s, 1H, two tautomeric forms), 12.65 and 12.70 (s, 1H, two tautomeric forms), 12.99 and 13.02 (s, 1H, two tautomeric forms)

### Example 19

### 2-(5-Benzyl-1,4,5,6-tetrahydro-pyrrolo[3,4-c]pyrazol-3-yl)-5-ethyl-7,7-dimethyl-5,7-dihydro-3H-imidazo[4,5-f]indol-6-one

In an analogous manner as described for example 13 2-(5-benzyl-1,4,5,6-tetrahydro-pyrrolo[3,4-c] pyrazol-3-yl)-5-ethyl-7,7-dimethyl-5,7-dihydro-3H-imidazo[4,5-f]indol-6-one was prepared from 5,6-diamino-1-ethyl-3,3-dimethyl-1,3-dihydro-indol-2-one (A1) and 5-benzyl-1,4,5,6-tetrahydro-pyrrolo[3,4-c]pyrazole-3-carboxylic acid ethyl ester (A9).
MS: M = 427.4 (ESI+)
¹H-NMR (400 MHz, DMSO): δ (ppm) = 1.09 (t, 3H), 1.29 (s, 6H), 3.74 (m, 2H), 3.87 (m, 4H), 3.99 (s, 2H), 6.96 and 6.25 (s, 1H, two tautomeric forms), 7.27 (m, 1H), 7.42 (m, 4H), 7.37 and 7.60 (s, 1H, two tautomeric forms), 12.60 (br, 1H), 12.95 and 13.25 (br, 1 H, two tautomeric forms)

### Example 20

### 3-(5-Ethyl-7,7-dimethyl-6-oxo-3,5,6,7-tetrahydro-imidazo [4,5-f]indol-2-yl)-4,6-dihydro-1H-pyrrolo[3,4-c]pyrazole-5-carboxylic acid (2,6-diethyl-phenyl)-amide

To a solution of 5-(2,6-diethyl-phenylcarbamoyl)-1,4,5,6-tetrahydro-pyrrolo[3,4-c]pyrazole-3-carboxylic acid (A11, 230mg, 0.700mmol) in absolute DMF (4ml) under a nitrogen atmosphere were added hydroxybenzotriazole hydrate (128.7mg, 0.841mmol), triethylamine (212.6mg, 293µl, 2.101mmol) and N'-(3-dimethylaminopropyl)-N-ethylcarbodiimide hydrochloride (161.1mg, 0.841mmol). After 2h at room temperature a solution of 5,6-diamino-1-ethyl-3,3-dimethyl-1,3-dihydro-indol-2-one (A1, 153.6mg, 0.700mmol) in DMF (2ml) was added and stirring continued at room temperature for 18h. Most of the DMF was evaporated and the reaction mixture was treated with water. The aqueous phase was extracted twice with ethyl acetate and the solvent of the combined organic phases was evaporated. The residue was dissolved in ethanol (7ml), treated with HCl (32%, 4ml) and heated under reflux for 3h. The solvent was evaporated and the residue alkalized with ammonia (25%). The aqueous phase was extracted three times with ethyl acetate, the combined organic phases were washed with brine, dried over MgSO₄ and the solvent was evaporated. The residue was purified by silica gel chromatography (CH₂Cl₂/ MeOH 95:5) to yield 163mg 3-(5-ethyl-7,7-dimethyl-6-oxo-3,5,6,7-tetrahydro-imidazo[4,5-f]indol-2-yl)-4,6-dihydro-1H-pyrrolo[3,4-c]pyrazole-5-carboxylic acid (2,6-diethyl-phenyl)-amide (0.319mmol, 45%).
MS: M = 512.3 (ESI+)
¹H-NMR (400 MHz, DMSO): δ (ppm) = 1.15 (m, 9H), 1.31 (s, 6H), 2.61 (q, 4H), 3.76 (q, 2H), 4.63 (s, 2H), 4.76 (s, 2H), 7.00 an 7.24 (s, 1H, two tautomeric forms), 7.10 (m, 2H), 7.18 (m, 1H), 7.40 and 7.60 (s, 1H, two tautomeric forms), 7.81 (br, 1H), 12.50 and 12.80 (br, 1H, two tautomeric forms), 13.25 and 13.50 (br, 1H, two tautomeric forms)

### Example 21

### 3-(5-Isopropyl-7,7-dimethyl-6-oxo-3,5,6,7-tetrahydro-imidazo [4,5-f]indol-2-yl)-4,6-dihydro-1H-pyrrolo[3,4-c]pyrazole-5-carboxylic acid (2,6-diethyl-phenyl)-amide

In an analogous manner as described for example 20 3-(5-Isopropyl-7,7-dimethyl-6-oxo-3,5,6,7-tetrahydro-imidazo[4,5-f]indol-2-yl)-4,6-dihydro-1H-pyrrolo[3,4-c]pyrazole-5-carboxylic acid (2,6-diethyl-phenyl)-amide was prepared from 5-(2,6-diethyl-phenylcarbamoyl)-1,4,5,6-tetrahydro-pyrrolo[3,4-c]pyrazole-3-carboxylic acid (A11) and 5,6-diamino-1-isopropyl-3,3-dimethyl-1,3-dihydro-indol-2-one (A4).
MS: M = 526.2 (ESI+)
¹H-NMR (400 MHz, DMSO): δ (ppm) = 1.15 (t, 6H), 1.30 (s, 6H), 1.45 (d, 6H), 2.61 (q, 4H), 4.58 (m, 1H), 4.63 (s, 2H), 4.76 (s, 2H), 7.10 (m, 2H), 7.15 (m, 1H), 7.30 and 7.40 (s, 1H, two tautomeric forms), 7.40 and 7.58 (s, 1H, two tautomeric forms), 7.81 (s, 1H), 12.65 and 12.78 (br, 1H, two tautomeric forms), 13.25 and 13.50 (br, 1H, two tautomeric forms)

### Example 22

### 2-(5-Cyclopropanecarbonyl-1,4,5,6-tetrahydro-pyrrolo[3,4-c]pyrazol-3-yl)-5-ethyl-7,7-dimethyl-5,7-dihydro-3H-imidazo[4,5-f]indol-6-one

In an analogous manner as described for example 20 2-(5-cyclopropanecarbonyl-1,4,5,6-tetrahydro-pyrrolo[3,4-c]pyrazol-3-yl)-5-ethyl-7,7-dimethyl-5,7-dihydro-3H-imidazo[4,5-f]indol-6-one was prepared from 5-cyclopropanecarbonyl-1,4,5,6-tetrahydro-pyrrolo[3,4-c]pyrazole-3-carboxylic acid (A12) and 5,6-diamino-1-ethyl-3,3-dimethyl-1,3-dihydro-indol-2-one (A1).
MS: M = 405.2 (API+)
¹H-NMR (400 MHz, DMSO): δ (ppm) = 0.84 (m, 4H), 1.19 (t, 3H), 1.32 (s, 6H), 1.91 (m, 1H), 3.78 (q, 2H), 4.55 and 4.65 (s, 2H, rotamers), 4.92 and 5.01 (s, 2H, rotamers), 7.05 and 7.26 (s, 1H, two tautomeric forms), 7.45 and 7.65 (s, 1H, two tautomeric forms), 12.75 (br, 1H), 13.30 and 13.55 (br, 1H, two tautomeric forms)

### Example 23

### 5-Ethyl-7,7-dimethyl-2-[5-(2-thiophen-2-yl-acetyl)-1,4,5,6-tetrahydro-pyrrolo[3,4-c]pyrazol-3-yl]-5,7-dihydro-3H-imidazo[4,5-f]indol-6-one

In an analogous manner as described for example 20 5-ethyl-7,7-dimethyl-2-[5-(2-thiophen-2-yl-acetyl)-1,4,5,6-tetrahydro-pyrrolo[3,4-c]pyrazol-3-yl]-5,7-dihydro-3H-imidazo[4,5-f]indol-6-one was prepared from 5-(2-thiophen-2-yl-acetyl)-1,4,5,6-tetrahydro-pyrrolo[3,4-c]pyrazole-3-carboxylic acid (A13) and 5,6-diamino-1-ethyl-3,3-dimethyl-1,3-dihydro-indol-2-one (A1).
MS: M = 461.1 (ESI+)
¹H-NMR (400 MHz, DMSO): δ (ppm) = 1.19 (t, 3H), 1.31 (s, 6H), 3.77 (q, 2H), 4.02 and 4.07 (s, 2H, rotamers), 4.51 - 4.98 (s, 4H, rotamers), 6.98 (m, 2H), 7.10 and 7.28 (s, 1H, two tautomeric forms), 7.40 (m, 1H), 7.50 and 7.62 (s, 1H, two tautomeric forms), 12.50 and 12.82 (1H, tautomeric forms and rotamers), 13.25 - 13.55 (1H, tautomeric forms and rotamers)

### Example 24

### 5-Isopropyl-7,7-dimethyl-2-[5-(2-thiophen-2-yl-acetyl)-1,4,5,6-tetrahydro-pyrrolo[3,4-c]pyrazol-3-yl]-5,7-dihydro-3H-imidazo[4,5-f]indol-6-one

In an analogous manner as described for example 20 5-Isopropyl-7,7-dimethyl-2-[5-(2-thiophen-2-yl-acetyl)-1,4,5,6-tetrahydro-pyrrolo[3,4-c]pyrazol-3-yl] -5,7-dihydro-3H-imidazo[4,5-f]indol-6-one was prepared from 5-(2-thiophen-2-yl-acetyl)-1,4,5,6-tetrahydro-pyrrolo[3,4-c]pyrazole-3-carboxylic acid (A13) and 5,6-diamino-1-isopropyl-3,3-dimethyl-1,3-dihydro-indol-2-one (A4).
MS: M = 475.1 (ESI+)
¹H-NMR (400 MHz, DMSO): δ (ppm) = 1.30 (s, 6H), 1.45 (d, 6H), 4.03 and 4.07 (s, 2H, rotamers), 4.51 - 4.98 (4H, rotamers), 4.58 (m, 1H), 6.98 (m, 2H), 7.10 - 7.30 (1H, tautomeric forms and rotamers), 7.40 (m, 1H), 7.50 - 7.65 (1H, tautomeric forms and rotamers), 12.40 -12.80 (1H, rotamers and tautomeric forms), 13.20 - 13.55 (1H, rotamers and tautomeric forms)

### Example 25

### 5-Ethyl-2-[5-(4-fluoro-benzenesulfonyl)-1,4,5,6-tetrahydro-pyrrolo[3,4-c]pyrazol-3-yl]-7,7-dimethyl-5,7-dihydro-3H-imidazo[4,5-f]indol-6-one

In an analogous manner as described for example 20 5-ethyl-2-[5-(4-fluoro-benzenesulfonyl)-1,4,5,6-tetrahydro-pyrrolo[3,4-c]pyrazol-3-yl]-7,7-dimethyl-5,7-dihydro-3H-imidazo[4,5-f]indol-6-one was prepared from 5-(4-fluoro-benzenesulfonyl)-1,4,5,6-tetrahydro-pyrrolo[3,4-c]pyrazole-3-carboxylic acid (A14) and 5,6-diamino-1-ethyl-3,3-dimethyl-1,3-dihydro-indol-2-one (A1).
MS: M = 495.2 (ESI+)
¹H-NMR (400 MHz, DMSO): δ (ppm) = 1.18 (t, 3H), 1.31 (s, 6H), 3.76 (q, 2H), 4.45 (s, 2H), 4.58 (s, 2H), 7.05 and 7.26 (s, 1H, two tautomeric forms), 7.47 (m, 2H), 7.26 and 7.62 (s, 1H, two tautomeric forms), 8.01 (m, 2H), 12.45 and 12.70 (br, 1H, two tautomeric forms), 13.20 and 13.52 (br, 1H, two tautomeric forms)

### Example 26

### 5-Ethyl-7,7-dimethyl-2-(1,4,6,7-tetrahydro-thiopyrano[4,3-c]pyrazol-3-yl)-5,7-dihydro-3H-imidazo[4,5-f]indol-6-one

In an analogous manner as described for example 20 5-ethyl-7,7-dimethyl-2-(1,4,6,7-tetrahydro-thiopyrano[4,3-c]pyrazol-3-yl)-5,7-dihydro-3H-imidazo[4,5-f]indol-6-one was prepared from 1,4,6,7-tetrahydro-thiopyrano[4,3-c]pyrazole-3-carboxylic acid (A15) and 5,6-diamino-1-ethyl-3,3-dimethyl-1,3-dihydro-indol-2-one (A1).
MS: M = 368.3 (ESI+)
¹H-NMR (400 MHz, DMSO): δ (ppm) = 1.19 (t, 3H), 1.30 (s, 6H), 2.92 (s, 4H), 3.75 (m, 2H), 3.99 (s, 2H), 6.97 and 7.29 (s, 1H, two tautomeric forms), 7.37 and 7.63 (s, 1H, two tautomeric forms), 12.63 and 12.68 (br, 1H, two tautomeric forms), 12.96 and 13.00 (br, 1H, two tautomeric forms)

### Example 27

### 2-(4,6-Dihydro-1H-thieno[3,4-c]pyrazol-3-yl)-5-ethyl-7,7-dimethyl-5,7-dihydro-3H-imidazo[4,5-f]indol-6-one

In an analogous manner as described for example 20 2-(4,6-dihydro-1H-thieno[3,4-c]pyrazol-3-yl)-5-ethyl-7,7-dimethyl-5,7-dihydro-3H-imidazo[4,5-f]indol-6-one was prepared from 4,6-dihydro-1H-thieno[3,4-c]pyrazole-3-carboxylic acid (A15) and 5,6-diamino-1-ethyl-3,3-dimethyl-1,3-dihydro-indol-2-one (A1).
MS: M = 354.2 (ESI+)
¹H-NMR (400 MHz, DMSO): δ (ppm) = 1.18 (t, 3H), 1.30 (s, 6H), 3.75 (q, 2H), 4.02 (s, 2H), 4.09 (s, 2H), 6.98 and 7.28 (s, 1H, two tautomeric forms), 7.38 and 7.65 (s, 1H, two tautomeric forms), 12.70 (br, 1H), 13.00 (br, 1H)

### Example 28

### 2-(4,6-Dihydro-1H-thieno[3,4-c]pyrazol-3-yl)-5-isopropyl-7,7-dimethyl-5,7-dihydro-3H-imidazo[4,5-f]indol-6-one

In an analogous manner as described for example 20 2-(4,6-dihydro-1H-thieno[3,4-c]pyrazol-3-yl)-5-isopropyl-7,7-dimethyl-5,7-dihydro-3H-imidazo[4,5-f]indol-6-one was prepared from 4,6-dihydro-1H-thieno[3,4-c]pyrazole-3-carboxylic acid (A15) and 5,6-diamino-1-isopropyl-3,3-dimethyl-1,3-dihydro-indol-2-one (A4).
MS: M = 368.1 (ESI+)
¹H-NMR (400 MHz, DMSO): δ (ppm) = 1.29 (s, 6H), 1.44 (d, 6H), 4.02 (s, 2H), 4.09 (s, 2H), 4.57 (m, 1H), 7.09 and 7.34 (s, 1H, two tautomeric forms), 7.34 and 7.63 (s, 1H, two tautomeric forms), 12.55 (br, 1H), 13.05 (br, 1H)

## Claims

1. A compound according to formula I, 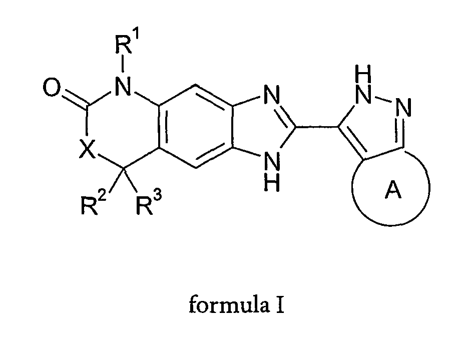 wherein,
R¹ is hydrogen;
alkyl, alkenyl or alkynyl,
wherein said alkyl, alkenyl or alkynyl is optionally substituted one or several times by nitro, cyano or -Y-R⁴;
Y is a single bond, -C(O)NH-, -C(O)N(alkyl)-, -N(alkyl)C(O)-, -NHC(O)-, -NHC(O)NH-, -NHC(O)N(alkyl)-, -NHS(O)₂-, -S(O)₂NH-, -S(O)₂N(alkyl)-, -S(O)₂-, -S(O)-, -C(O)O-, -OC(O)-, -C(O)-, -P(O)(alkyl)-, -NH-, -N(alkyl)-, -O- or -S-;
R⁴ is alkyl, wherein said alkyl is optionally substituted one or several times by halogen, hydroxy, alkoxy, alkoxyalkoxy, amino, alkylamino, dialkylamino, -C(O)OH or -C(O)NH₂;
aryl, wherein the aryl is optionally substituted one or several times by halogen, cyano, nitro, amino, hydroxy, (C₁-C₄)alkyl, (C₁-C₄)alkoxy, halogenated (C₁-C₄)alkyl or halogenated (C₁-C₄)alkoxy;
heteroaryl, wherein the heteroaryl is optionally substituted one or several times by alkyl;
cycloalkyl; or
heterocyclyl;
R² is hydrogen or alkyl;
R³ is hydrogen or alkyl;
or alternatively R² and R³ form together with the carbon atom to which they are attached a (C₅-C₆)cycloalkyl ring;
X is a single bond, -CH₂- or -C(alkyl)₂-;
ring A is a 5 to 7 membered saturated ring optionally containing one or two heteroatoms independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-alkyl,
-C(O)-cycloalkyl,
-C(O)-heteroryclyl,
-C(O)-(CH₂)ₙ-phenyl, wherein the phenyl is optionally substituted once or twice with halogen, alkyl, alkoxy, nitro, amino, alkylamino, dialkylamino, cyano, trifluoromethyl or trifluoromethoxy,
-C(O)-(CH₂)ₙ-heteroaryl,
-C(O)-NH-phenyl, wherein the phenyl is optionally substituted once or twice with halogen, alkyl, alkoxy, nitro, amino, alkylamino, dialkylamino, cyano, trifluoromethyl or trifluoromethoxy, or
-S(O)₂-phenyl, wherein the phenyl is optionally substituted once or twice with halogen, alkyl, alkoxy, nitro, amino, alkylamino, dialkylamino, cyano, trifluoromethyl or trifluoromethoxy;
n is 0, 1 or 2;
and all pharmaceutically acceptable salts thereof.

2. The compounds according to claim 1, wherein
ring A is a 5 to 7 membered saturated ring optionally containing one or two heteroatoms independently selected from oxygen, nitrogen or sulfur and the remaining ring atoms being carbon atoms, wherein said ring A can be optionally substituted one or several times by alkyl.

3. The compounds according to claim 1, wherein
R¹ is hydrogen or alkyl,
wherein said alkyl is optionally substituted once by -Y-R⁴;
Y is a single bond;
R⁴ is heterocyclyl;
R² is alkyl;
R³ is alkyl;
X is a single bond;
ring A is a 5 to 7 membered saturated ring optionally containing one or two heteroatoms independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O) -cycloalkyl,
-C(O)-heterocyclyl,
-C(O)-(CH₂)ₙ-phenyl, wherein the phenyl is optionally substituted once or twice with halogen,
-C(O)-(CH₂)ₙ-heteroaryl,
-C(O)-NH-phenyl, wherein the phenyl is optionally substituted once or twice with alkyl, or
-S(O)₂-phenyl, wherein the phenyl is optionally substituted once or twice with halogen; and
n is 0 or 1.

4. The compounds according to claim 1, wherein
R¹, R² and R³ are alkyl;
X is a single bond;
ring A is a 5 to 7 membered, preferably 5 to 6 membered, saturated ring optionally containing one heteroatom independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-cycloalkyl,
-C(O)-heterocyclyl,
-C(O)-(CH₂)ₙ-phenyl, wherein the phenyl is optionally substituted once or twice with halogen,
-C(O)-(CH₂)ₙ-thienyl,
-C(O)-NH-phenyl, wherein the phenyl is optionally substituted once or twice with alkyl, or
-S(O)₂-phenyl, wherein the phenyl is optionally substituted once or twice with halogen; and
n is 0 or 1.

5. The compounds according to claim 1, wherein
R¹ is hydrogen or alkyl,
wherein said alkyl is optionally substituted once by -Y-R⁴;
Y is a single bond;
R⁴ is heterocyclyl;
R² is alkyl;
R³ is alkyl;
X is a single bond; and
ring A is a 5 to 7 membered saturated hydrocarbon ring.

6. The compounds according to claim 5, selected from the group consisting of:
7,7-Dimethyl-2-(1,4,5,6-tetrahydro-cyclopentapyrazol-3-yl)-5,7-dihydro-3H-imidazo[4,5-f]indol-6-one;
7,7-Dimethyl-2-(4,5,6,7-tetrahydro-1H-indazol-3-yl)-5,7-dihydro-3H-imidazo[4,5-f]indol-6-one;
5,7,7-Trimethyl-2-(1,4,5,6-tetrahydro-cyclopentapyrazol-3-yl)-5,7-dihydro-3H-imidazo [4,5-f]indol-6-one;
5-Ethyl-7,7-dimethyl-2-(1,4,5,6-tetrahydro-cyclopentapyrazol-3-yl)-5,7-dihydro-3H-imidazo[4,5-f]indol-6-one;
7,7-Dimethyl-5-propyl-2-(1,4,5,6-tetrahydro-cyclopentapyrazol-3-yl)-5,7-dihydro-3H-imidazo[4,5-f] indol-6-one;
5-Isopropyl-7,7-dimethyl-2-(1,4,5,6-tetrahydro-cyclopentapyrazol-3-yl)-5,7-dihydro-3H-imidazo[4,5-f]indol-6-one; and
7,7-Dimethyl-5-(3-morpholin-4-yl-propyl)-2-(1,4,5,6-tetrahydro-cyclopentapyrazol-3-yl)-5,7-dihydro-3H-imidazo[4,5-f] indol-6-one;
5-Ethyl-7,7-dimethyl-2-(4,5,6,7-tetrahydro-1H-indazol-3-yl)-5,7-dihydro-3H-imidazo[4,5-f]indol-6-one;
5-Isopropyl-7,7-dimethyl-2-(4,5,6,7-tetrahydro-1H-indazol-3-yl)-5,7-dihydro-3H-imidazo[4,5-f]indol-6-one;
7,7-Dimethyl-5-(3-morpholin-4-yl-propyl)-2-(4,5,6,7-tetrahydro-1H-indazol-3-yl)-5,7-dihydro-3H-imidazo[4,5-f]indol-6-one; and
5,7,7-Triethyl-2-(4,5,6,7-tetrahydro-1H-indazol-3-yl)-5,7-dihydro-3H-imidazo[4,5-f]indol-6-one.

7. The compounds according to claim 1, wherein
R¹, R² and R³ are alkyl;
X is a single bond; and
ring A is a 5 to 7 membered saturated ring containing one heteroatom independently selected from oxygen, nitrogen or sulfur and the remaining ring atoms being carbon atoms,
wherein said ring A can be optionally substituted one or several times by alkyl,
and if said ring A contains one nitrogen, said nitrogen can be optionally substituted once by
-CH₂-phenyl,
-C(O)-cycloalkyl,
-C(O)-heterocyclyl,
-C(O)-(CH₂)ₙ-phenyl, wherein the phenyl is optionally substituted once or twice with halogen,
-C(O)-(CH₂)ₙ-thienyl,
-C(O)-NH-phenyl, wherein the phenyl is optionally substituted once or twice with alkyl, or
-S(O)₂-phenyl, wherein the phenyl is optionally substituted once or twice with halogen; and
n is 0 or 1;

8. The compounds according to claim 7, selected from the group consisting of:
5-Ethyl-7,7-dimethyl-2-(1,4,6,7-tetrahydro-pyrano[4,3-*c*]pyrazol-3-yl)-5,7-dihydro-3H-imidazo [4,5-f] indol-6-one;
2-(5-Benzyl-4,5,6,7-tetrahydro-1*H*-pyrazolo[4,3-*c*] pyridin-3-yl)-5-ethyl-7,7-dimethyl-5,7-dihydro-3*H*-imidazo[4,5-*f*]indol-6-one;
5-Ethyl-7,7-dimethyl-2-(4,5,6,7-tetrahydro-1*H*-pyrazolo[4,3-*c*]pyridin-3-yl)-5,7-dihydro-3*H*-imidazo[4,5-*f*]indol-6-one;
2-(5-Benzyl-1,4,5,6-tetrahydro-pyrrolo [3,4-*c*] pyrazol-3-yl)-5-ethyl-7,7-dimethyl-5,7-dihydro-3*H*-imidazo[4,5-*f*]indol-6-one;
5-Ethyl-7,7-dimethyl-2-(1,4,6,7-tetrahydro-thiopyrano [4,3-*c*] pyrazol-3-yl)-5,7-dihydro-3*H*-imidazo[4,5-*f*] indol-6-one;
2-(5-Cyclopropanecarbonyl-1,4,5,6-tetrahydro-pyrrolo [3,4-*c*]pyrazol-3-yl)-5-ethyl-7,7-dimethyl-5,7-dihydro-3*H*-imidazo[4-5-*f*]indol-6-one;
5-Ethyl-2-{5-[2-(4-fluoro-phenyl)-acetyl] -4,5,6,7-tetrahydro-1*H-*pyrazolo[4,3-*c*]*pyridin-3-yl}-7,7-dimethyl-5,7-dihydro-3H*-imidazo[4,5-*f*]indol-6-one;
5-Ethyl-2-[5-(4-fluoro-benzenesulfonyl)-4,5,6,7-tetrahydro-1*H-*pyrazolo[4,3-*c*]pyridin-3-yl]-7,7-dimethyl-5,7-dihydro-3*H*-imidazo[4,5-*f*]indol-6-one;
5-Ethyl-7,7-dimethyl-2-[5-(morpholine-4-carbonyl)-4,5,6,7-tetrahydro-1*H-*pyrazolo[4,3-*c*]pyridin-3-yl]-5,7-dihydro-3*H*-imidazo[4,5-*f*]indol-6-one;
5-Ethyl-2-[5-(4-fluoro-benzoyl)-4,5,6,7-tetrahydro-1*H*-pyrazolo[4,3-*c*]pyridin-3-yl]-7,7-dimethyl-5,7-dihydro-3*H*-imidazo[4,5-*f*]indol-6-one;
2-(4,6-Dihydro-1*H*-thieno[3,4-*c*]pyrazol-3-yl)-5-ethyl-7,7-dimethyl-5,7-dihydro-3*H*-imidazo[4,5-*f*]indol-6-one;
5-Ethyl-7,7-dimethyl-2-[5-(2-thiophen-2-yl-acetyl)-1,4,5,6-tetrahydro-pyrrolo[3,4-*c*]pyrazol-3-yl]-5,7-dihydro-3*H*-imidazo[4,5-*f*]indol-6-one;
2-(4,6-Dihydro-1*H*-thieno[3,4-*c*]pyrazol-3-yl)-5-isopropyl-7,7-dimethyl-5,7-dihydro-3*H*-imidazo[4,5-*f*]indol-6-one;
3-(5-Ethyl-7,7-dimethyl-6-oxo-3,5,6,7-tetrahydro-imidazo[4,5-*f*]indol-2-yl)-4,6-dihydro-1*H*-pyrrolo[3,4-*c*]pyrazole-5-carboxylic acid (2,6-diethylphenyl)-amide;
5-Ethyl-2-[5-(4-fluoro-benzenesulfonyl)-1,4,5,6-tetrahydro-pyrrolo[3,4-*c*]pyrazol-3-yl]-7,7-dimethyl-5,7-dihydro-3*H*-imidazo[4,5-*f*]indol-6-one;
3-(5-Isopropyl-7,7-dimethyl-6-oxo-3,5,6,7-tetrahydro-imidazo[4,5-*f*]indol-2-yl)-4,6-dihydro-1*H*-pyrrolo[3,4-*c*]pyrazole-5-carboxylic acid (2,6-diethylphenyl)-amide; and
5-Isopropyl-7,7-dimethyl-2-[5-(2-thiophen-2-yl-acetyl)-1,4,5,6-tetrahydro-pyrrolo[3,4-*c*]pyrazol-3-yl]-5,7-dihydro-3*H*-imidazo[4,5-*f*]indol-6-one.

9. A process for the preparation of the compounds of formula I according to claim 1 comprising the steps of
a. reacting a compounds of formula II, 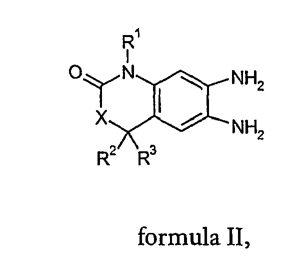 wherein X, R¹, R² and R³ have the significance given above in claim 1, with a compound of formula III, 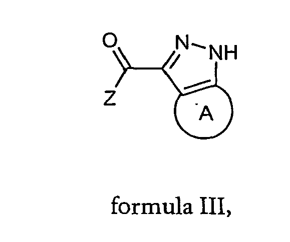 wherein ring A has the significance given above in claim 1 and Z is -OH, -Cl, -H, -OMe or hydroxybenzotriazole,
to give the compounds of formula I 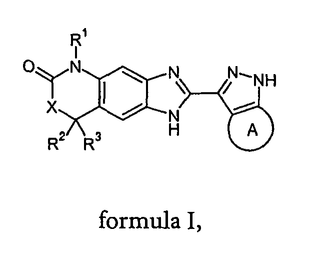
b. isolating the compounds of formula I; and
c. if desired, converting the compounds of formula I into their pharmaceutically acceptable salts

10. A pharmaceutical composition, containing one or more compounds according to claims 1 to 8, together with pharmaceutically acceptable excipients.

11. A pharmaceutical composition, containing one or more compounds according to claims 1 to 8, for the inhibition of tumor growth.

12. The use of a compound according to claims 1 to 8, for the manufacture of corresponding medicaments for the inhibition of tumor growth.

## Patentansprüche

1. Verbindung nach Formel I, 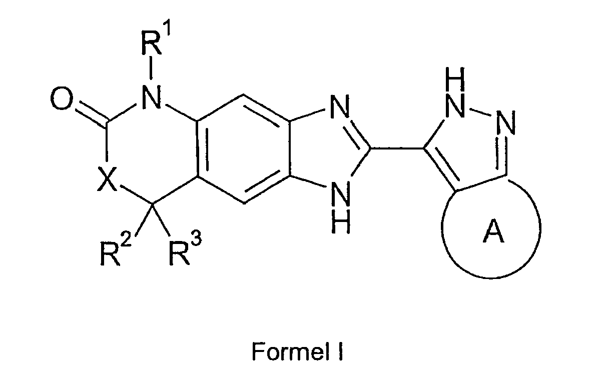 wobei
R¹ Wasserstoff ist;
Alkyl, Alkenyl oder Alkinyl,
wobei das Alkyl, Alkenyl oder Alkinyl optional ein- oder mehrfach durch Nitro, Cyano oder -Y R⁴ substituiert ist;
Y eine Einfachbindung, -C(O)NH-, -C(O)N(Alkyl)-, -N(Alkyl)C(O)-, -NHC(O)-, -NHC(O)NH-, -NHC(O)N(Alkyl)-, -NHS(O)₂-, -S(O)₂NH-, -S(O)₂N(Alkyl)-, -S(O)₂-, -S(O)-, -C(O)O-, -OC(O)-, -C(O)-, -P(O) (Alkyl)-, -NH-, -N(Alkyl)-, -O- oder -S- ist;
R⁴ Alkyl ist, wobei das Alkyl optional ein- oder mehrfach durch Halogen, Hydroxy, Alkoxy, Alkoxyalkoxy, Amino, Alkylamino, Dialkylamino, -C(O)OH oder -C(O)NH₂ substituiert ist;
Aryl, wobei das Aryl optional ein- oder mehrfach durch Halogen, Cyano, Nitro, Amino, Hydroxy, (C₁-C₄)Alkyl, (C₁-C₄)Alkoxy, halogeniertes (C₁-C₄)Alkyl oder halogeniertes (C₁-C₄)Alkoxy substituiert ist;
Heteroaryl, wobei das Heteroaryl optional ein- oder mehrfach durch Alkyl substituiert ist;
Cycloalkyl; oder
Heterocyclyl;
R² Wasserstoff oder Alkyl ist;
R³ Wasserstoff oder Alkyl ist;
oder alternativ R² und R³ zusammen mit dem Kohlenstoffatom, an das sie gebunden sind, einen (C₅-C₆)Cycloalkylring bilden;
X eine Einfachbindung, -CH₂- oder -C(Alkyl)₂- ist;
Ring A ein 5- bis 7-gliedriger gesättigter Ring ist, optional enthaltend ein oder zwei Heteroatome unabhängig ausgewählt aus Sauerstoff, Stickstoff oder Schwefel und wobei die übrigen Ringatome Kohlenstoffatome sind, wobei der Ring A optional ein- oder mehrfach durch Alkyl substituiert sein kann,
und, wenn der Ring A einen Stickstoff enthält, der Stickstoff optional einfach substituiert sein kann durch
-CH₂-Phenyl,
-C(O)-Alkyl,
-C(O)-Cycloalkyl,
-C(O)-Heterocyclyl,
-C(O)-(CH₂)ₙ-Phenyl, wobei das Phenyl optional ein- oder zweifach durch Halogen, Alkyl, Alkoxy, Nitro, Amino, Alkylamino, Dialkylamino, Cyano, Trifluormethyl oder Trifluormethoxy substituiert sein kann,
-C(O)-(CH₂)ₙ-Heteroaryl,
-C(O)-NH-Phenyl, wobei das Phenyl optional ein- oder zweifach durch Halogen, Alkyl, Alkoxy, Nitro, Amino, Alkylamino, Dialkylamino, Cyano, Trifluormethyl oder Trifluormethoxy substituiert ist, oder
-S(O)₂-Phenyl, wobei das Phenyl optional ein- oder zweifach durch Halogen, Alkyl, Alkoxy, Nitro, Amino, Alkylamino, Dialkylamino, Cyano, Trifluormethyl oder Trifluormethoxy substituiert ist;
n 0, 1 oder 2 ist;
und alle pharmazeutisch akzeptablen Salze davon.

2. Die Verbindungen nach Anspruch 1, wobei
Ring A ein 5- bis 7-gliedriger gesättigter Ring ist, optional enthaltend ein oder zwei Heteroatome unabhängig ausgewählt aus Sauerstoff, Stickstoff oder Schwefel und wobei die übrigen Ringatome Kohlenstoffatome sind, wobei der Ring A optional ein- oder mehrfach durch Alkyl substituiert sein kann.

3. Die Verbindungen nach Anspruch 1, wobei
R¹ Wasserstoff oder Alkyl ist, wobei das Alkyl optional einfach mit -Y-R⁴ substituiert ist;
Y eine Einfachbindung ist;
R⁴ Heterocyclyl ist;
R² Alkyl ist;
R³ Alkyl ist;
X eine Einfachbindung ist;
Ring A ein 5- bis 7-gliedriger gesättigter Ring ist, optional enthaltend ein oder zwei Heteroatome unabhängig ausgewählt aus Sauerstoff, Stickstoff oder Schwefel und wobei die übrigen Ringatome Kohlenstoffatome sind, wobei der Ring A optional ein- oder mehrfach durch Alkyl substituiert sein kann,
und, wenn der Ring A einen Stickstoff enthält, der Stickstoff optional einfach substituiert sein kann durch
-CH₂-Phenyl,
-C(O)-Cycloalkyl,
-C(O)-Heterocyclyl,
-C(O)-(CH₂)ₙ-Phenyl, wobei das Phenyl optional ein- oder zweifach durch Halogen substituiert ist,
-C(O)-(CH₂)ₙ-Heteroaryl,
-C(O)-NH-Phenyl, wobei das Phenyl optional ein- oder zweifach durch Alkyl substituiert ist, oder
-S(O)₂-Phenyl, wobei das Phenyl optional ein- oder zweifach durch Halogen substituiert ist; und
n 0 oder 1 ist.

4. Die Verbindungen nach Anspruch 1, wobei
R¹, R² und R³ Alkyl sind;
X eine Einfachbindung ist;
Ring A ein 5- bis 7-gliedriger, bevorzugt 5- bis 6-gliedriger, gesättigter Ring ist, optional enthaltend ein Heteroatom unabhängig ausgewählt aus Sauerstoff, Stickstoff, Schwefel und wobei die übrigen Ringatome Kohlenstoffatome sind, wobei der Ring A optional ein- oder mehrfach durch Alkyl substituiert sein kann und, wenn der Ring A einen Stickstoff enthält, der Stickstoff optional einfach substituiert sein kann durch
-CH₂-Phenyl,
-C(O)-Cycloalkyl,
-C(O)-Heterocyclyl,
-C(O)-(CH₂)ₙ-Phenyl, wobei das Phenyl optional ein- oder zweifach durch Halogen substituiert ist,
-C(O)-(CH₂)ₙ- Thienyl,
-C(O)-NH-Phenyl, wobei das Phenyl optional ein- oder zweifach durch Alkyl substituiert ist, oder
-S(O)₂-Phenyl, wobei das Phenyl optional ein- oder zweifach durch Halogen substituiert ist; und
n 0 oder 1 ist.

5. Die Verbindungen nach Anspruch 1, wobei R¹ Wasserstoff oder Alkyl ist, wobei das Alkyl optional einfach durch -Y-R⁴ substituiert ist;
Y eine Einfachbindung ist;
R⁴ Heterocyclyl ist;
R² Alkyl ist;
R³ Alkyl ist;
X eine Einfachbindung ist; und
Ring A ein 5- bis 7-gliedriger gesättigter Kohlenwasserstoffring ist.

6. Die Verbindungen nach Anspruch 5, ausgewählt aus der Gruppe bestehend aus:
7,7-Dimethyl-2-(1,4,5,6-tetrahydro-cyclopentapyrazol-3-yl)-5,7-dihydro-3H-imidazo[4,5-f]indol-6-on;
7,7-Dimethyl-2-(4,5,6,7-tetrahydro-1H-indazol-3-yl)-5,7-dihydro-3H-imidazo[4,5-f]indol-6-on;
5,7,7-Trimethyl-2-(1,4,5,6-tetrahydro-cyclopentapyrazol-3-yl)-5,7-dihydro-3H-imidazo[4,5-f] indol-6-on;
5-Ethyl-7,7-dimethyl-2-(1,4,5,6-tetrahydro-cyclopentapyrazol-3-yl)-5,7-dihydro-3H-imidazo[4,5-f]indol-6-on;
7,7-Dimethyl-5-propyl-2-(1,4,5,6-tetrahydro-cyclopentapyrazol-3-yl)-5,7-dihydro-3H-imidazo[4,5-f]indol-6-on;
5-Isopropyl-7,7-dimethyl-2-(1,4,5,6-tetrahydro-cyclopentapyrazol-3-yl)-5,7-dihydro-3H-imidazo[4,5-f]indol-6-on; und
7,7-Dimethyl-5-(3-morpholin-4-yl-propyl)-2-(1,4,5,6-tetrahydro-cyclopentapyrazol-3-yl)-5,7-dihydro-3H-imidazo[4,5-f]indol-6-on;
5-Ethyl-7,7-dimethyl-2-(4,5,6,7-tetrahydro-1H-indazol-3-yl)-5,7-dihydro-3H-imidazo[4,5-f]indol-6-on;
5-Isopropyl-7,7-dimethyl-2-(4,5,6,7-tetrahydro-1H-indazol-3-yl)-5,7-dihydro-3H-imidazo[4,5-f]indol-6-on;
7,7-Dimethy(-5-(3-morpholin-4-yl-propyl)-2-(4,5,6,7-tetrahydro-1H-indazol-3-yl)-5,7-dihydro-3H-imidazo[4,5-f]indol-6-on; und
5,7,7-Triethyl-2-(4,5,6,7-tetrahydro-1H-indazol-3-yl)-5,7-dihydro-3H-imidazo[4,5-f]indol-6-on.

7. Die Verbindungen nach Anspruch 1, wobei
R¹, R² und R³ Alkyl sind;
X eine Einfachbindung ist;
Ring A ein 5- bis 7-gliedriger, gesättigter Ring ist, enthaltend ein Heteroatom unabhängig ausgewählt aus Sauerstoff, Stickstoff oder Schwefel und wobei die übrigen Ringatome Kohlenstoffatome sind, wobei der Ring A optional ein- oder mehrfach durch Alkyl substituiert sein kann und, wenn der Ring A einen Stickstoff enthält, der Stickstoff optional einfach substituiert sein kann durch
-CH₂-Phenyl,
-C(O)-Cycloalkyl,
-C(O)-Heterocyclyl,
-C(O)-(CH₂)ₙ-Phenyl, wobei das Phenyl optional ein- oder zweifach durch Halogen substituiert ist,
-C(O)-(CH₂)ₙ-Thienyl,
-C(O)-NH-Phenyl, wobei das Phenyl optional ein- oder zweifach durch Alkyl substituiert ist, oder
-S(O)₂-Phenyl, wobei das Phenyl optional ein- oder zweifach durch Halogen substituiert ist; und
n 0 oder 1 ist.

8. Die Verbindungen nach Anspruch 7, ausgewählt aus der Gruppe bestehend aus:
5-Ethyl-7,7-dimethyl-2-(1,4,6,7-tetrahydro-pyrano[4,3-c]pyrazol-3-yl)-5,7-dihydro-3H-imidazo[4,5-f]indol-6-on;
2-(5-Benzyl-4,5,6,7-tetrahydro-1 H-pyrazolo[4,3-c]pyridin-3-yl)-5-ethyl-7,7-dimethyl-5,7-dihydro-3H-imidazo[4,5-f]indol-6-on;
5-Ethyl-7,7-dimethyl-2-(4,5,6,7-tetrahydro-1H-pyrazolo[4,3-c]pyridin-3-yl)-5,7-dihydro-3H-imidazo[4,5-f]indol-6-on;
2-(5-Benzyl-1,4,5,6-tetrahydro-pyrrolo[3,4-c]pyrazol-3-yl)-5-ethyl-7,7-dimethyl-5,7-dihydro-3H-imidazo[4,5-f]indol-6-on;
5-Ethyl-7,7-dimethyl-2-(1,4,6,7-tetrahydro-thiopyrano[4,3-c]pyrazol-3-yl)-5,7-dihydro-3H-imidazo[4,5-f]indol-6-on;
2-(5-Cyclopropancarbonyl-1,4,5,6-tetrahydro-pyrrolo[3,4-c]pyrazol-3-yl)-5-ethyl-7,7-dimethyl-5,7-dihydro-3H-imidazo[4,5-f]indol-6-on;
5-Ethyl-2-{5-[2-(4-fluor-phenyl)-acetyl]-4,5,6,7-tetrahydro-1H-pyrazolo[4,3-c]pyridin-3-yl}-7,7-dimethyl-5,7-dihydro-3H-imidazo[4,5-f]indol-6-on;
5-Ethyl-2-[5-(4-fluor-benzolsulfonyl)-4,5,6,7-tetrahydro-1H-pyrazolo[4,3-c]pyridin-3-yl]-7,7-dimethyl-5,7-dihydro-3H-imidazo[4,5-f]indol-6-on;
5-Ethyl-7,7-dimethyl-2-[5-(morpholin-4-carbonyl)-4,5,6,7-tetrahydro-1H-pyrazolo[4,3-c]pyridin-3-yl]-5,7-dihydro-3H-imidazo[4,5-f]indol-6-on;
5-Ethyl-2-[5-(4-fluor-benzoyl)-4,5,6,7-tetrahydro-1 H-pyrazolo[4,3-c]pyridin-3-yl]-7,7-dimethyl-5,7-dihydro-3H-imidazo[4,5-f]indol-6-on;
2-(4,6-Dihydro-1 H-thieno[3,4-c]pyrazol-3-yl)-5-ethyl-7,7-dimethyl-5,7-dihydro-3H-imidazo[4,5-f]indol-6-on;
5-Ethyl-7,7-dimethyl-2-[5-(2-thiophen-2-yl-acetyl)-1,4,5,6-tetrahydro-pyrrolo[3,4-c]pyrazol-3-yl]-5,7-dihydro-3H-imidazo[4,5-f]indol-6-on;
2-(4,6-Dihydro-1H-thieno[3,4-c]pyrazol-3-yl)-5-isopropyl-7,7-dimethyl-5,7-dihydro-3H-imidazo[4,5-f]indol-6-on;
3-(5-Ethyl-7,7-dimethyl-6-oxo-3,5,6,7-tetrahydro-imidazo[4,5-f]indol-2-yl)-4,6-dihydro-1 H-pyrrolo[3,4-c]pyrazol-5-carbonsäure(2,6-diethyl-phenyl)-amid;
5-Ethyl-2-[5-(4-fluor-benzolsulfonyl)-1,4,5,6-tetrahydro-pyrrolo[3,4-c]pyrazol-3-yl]-7,7-dimethyl-5,7-dihydro-3H-imidazo[4,5-f]indol-6-on;
3-(5-Isopropyl-7,7-dimethyl-6-oxo-3,5,6,7-tetrahydro-imidazo[4,5-f]indol-2-yl)-4,6-dihydro-1H-pyrrolo[3,4-c]pyrazol-5-carboxylic acid (2,6-diethyl-phenyl)-amid; und
5-Isopropyl-7,7-dimethyl-2-[5-(2-thiophen-2-yl-acetyl)-1,4,5,6-tetrahydro-pyrrolo[3,4-c]pyrazol-3-yl]-5,7-dihydro-3H-imidazo[4,5-f]indol-6-on.

9. Verfahren zur Herstellung der Verbindungen der Formel I nach Anspruch 1, umfassend die Schritte
a. Umsetzen einer Verbindung der Formel II 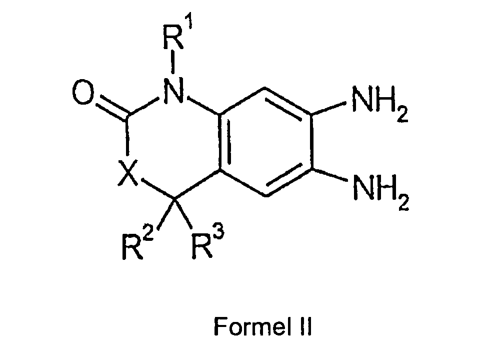 wobei X, R¹, R² und R³ die oben in Anspruch 1 gegebene Bedeutung haben, mit einer Verbindung der Formel III 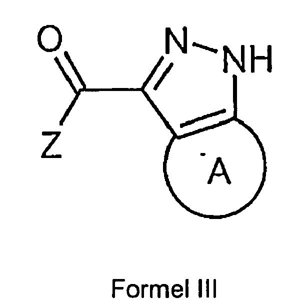 wobei Ring A die oben in Anspruch 1 gegebene Bedeutung hat und Z -OH, -Cl, -H, -OMe oder Hydroxybenzotriazol ist,
um die Verbindungen der Formel I zu ergeben 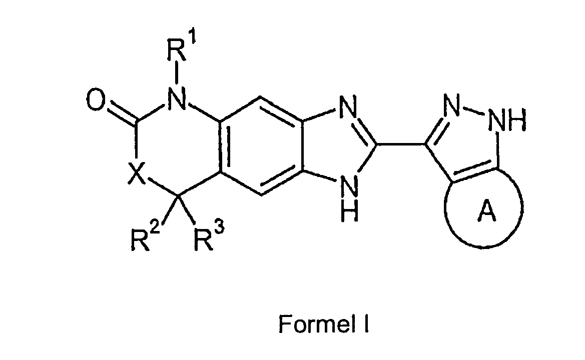
b. Isolieren der Verbindungen der Formel I; und
c. gegebenenfalls die Verbindungen der Formel I in ihre pharmazeutisch akzeptablen Salze umwandeln.

10. Pharmazeutische Zusammensetzung, enthaltend eine oder mehrere Verbindungen nach den Ansprüchen 1 bis 8, zusammen mit pharmazeutisch akzeptablen Hilfsstoffen.

11. Pharmazeutische Zusammensetzung, enthaltend eine oder mehrere Verbindungen nach den Ansprüchen 1 bis 8, zur Hemmung von Tumorwachstum.

12. Die Verwendung einer Verbindung nach den Ansprüchen 1 bis 8 zur Herstellung entsprechender Medikamente zur Hemmung von Tumorwachstum.

## Revendications

1. Composé selon la formule I, 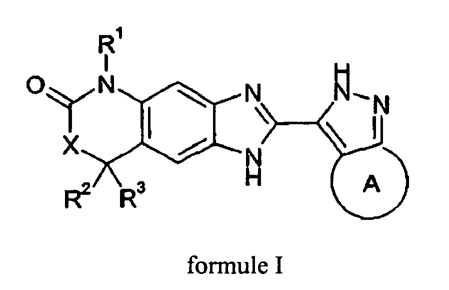 dans laquelle,
R¹ est un atome d'hydrogène ;
un groupe alkyle, alcényle ou alcynyle,
dans lequel ledit groupe alkyle, alcényle ou alcynyle est facultativement substitué une ou plusieurs fois par un groupe nitro, cyano ou -Y-R⁴ ;
Y est une simple liaison, -C(O)NH-, -C(O)N(alkyle)-, -N-(alkyl)C(O)-, -NHC(O)-, -NHC(O)NH-, -NHC(O)N(alkyle)-, -NHS(O)₂-, -S(O)₂NH-, S(O)₂N(alkyle)-, -S(O)₂-, -S(O)-, -C(O)O-, -OC(O)-, -C(O)-, -P(O)(alkyle)-, -NH-, -N-(alkyle)-, -O- ou -S- ;
R⁴ est un groupe alkyle, dans lequel ledit groupe alkyle est facultativement substitué une ou plusieurs fois par un atome d'halogène, un groupe hydroxy, alcoxy, alcoxyalcoxy, amino, alkylamino, dialkylamino, -C(O)OH ou -C(O)NH₂;
un groupe aryle, dans lequel le groupe aryle est facultativement substitué une ou plusieurs fois par un atome d'halogène, un groupe cyano, nitro, amino, hydroxy, alkyle en C₁ à C₄, alcoxy en C₁ à C₄, alkyle halogéné en C₁ à C₄ ou alcoxy halogéné en C₁ à C₄ ;
un groupe hétéroaryle, dans lequel le groupe hétéroaryle est facultativement substitué une ou plusieurs fois par un groupe alkyle ;
un groupe cycloalkyle ; ou
un groupe hétérocyclyle ;
R² est un atome d'hydrogène ou un groupe alkyle ;
R³ est un atome d'hydrogène ou un groupe alkyle ;
ou en variante R² et R³ forment ensemble avec l'atome de carbone auquel ils sont fixés un cycle cycloalkyle en C₅ à C₆ ;
X est une simple liaison, -CH₂- ou -C(alkyle)₂- ;
le cycle A est un cycle saturé à 5 à 7 chaînons contenant facultativement un ou deux hétéroatomes indépendamment choisis parmi un atome d'oxygène, d'azote ou de soufre et les atomes de cycle restants étant des atomes de carbone,
dans lequel ledit cycle A peut être facultativement substitué une ou plusieurs fois par un groupe alkyle,
et si ledit cycle A contient un atome d'azote, ledit atome d'azote peut être facultativement substitué une fois par
un groupe -CH₂-phényle,
un groupe -C(O)-alkyle,
un groupe -C(O)-cycloalkyle,
un groupe -C(O)-hétérocyclyle,
un groupe -C(O)-(CH₂)ₙ-phényle, dans lequel le groupe phényle est facultativement substitué une ou deux fois par un atome d'halogène, un groupe alkyle, alcoxy, nitro, amino, alkylamino, dialkylamino, cyano, trifluorométhyle ou trifluorométhoxy,
un groupe -C(O)-(CH₂)ₙ-hétéroaryle,
un groupe -C(O)-NH-phényle, dans lequel le groupe phényle est facultativement substitué une ou deux fois par un atome d'halogène, un groupe alkyle, alcoxy, nitro, amino, alkylamino, dialkylamino, cyano, trifluorométhyle ou trifluorométhoxy, ou
un groupe -S(O)₂-phényle, dans lequel le groupe phényle est facultativement substitué une ou deux fois par un atome d'halogène, un groupe alkyle, alcoxy, nitro, amino, alkylamino, dialkylamino, cyano, trifluorométhyle ou trifluorométhoxy ;
n vaut 0, 1 ou 2 ;
et tous les sels pharmaceutiquement acceptables de celui-ci.

2. Composés selon la revendication 1, dans lesquels
le cycle A est un cycle saturé à 5 à 7 chaînons contenant facultativement un ou deux hétéroatomes indépendamment choisis parmi un atome d'oxygène, un atome d'azote ou un atome de soufre et les atomes de cycle restants étant des atomes de carbone, dans lesquels ledit cycle A peut être facultativement substitué une ou plusieurs fois par un groupe alkyle.

3. Composés selon la revendication 1, dans lesquels
R¹ est un atome d'hydrogène ou un groupe alkyle,
dans lequel ledit groupe alkyle est facultativement substitué une fois par -Y-R⁴ ;
Y est une simple liaison ;
R⁴ est un groupe hétérocyclyle ;
R² est un groupe alkyle ;
R³ est un groupe alkyle ;
X est une simple liaison ;
le cycle A est un cycle saturé à 5 à 7 chaînons contenant facultativement un ou deux hétéroatomes indépendamment choisis parmi un atome d'oxygène, un atome d'azote ou un atome de soufre et les atomes de cycle restants étant des atomes de carbone,
dans lequel ledit cycle A peut être facultativement substitué une ou plusieurs fois par un groupe alkyle,
et si ledit cycle A contient un atome d'azote, ledit atome d'azote peut être facultativement substitué une fois par
un groupe -CH₂-phényle,
un groupe -C(O)-cycloalkyle,
un groupe -C(O)-hétérocyclyle,
un groupe -C(O)-(CH₂)ₙ-phényle, dans lequel le groupe phényle est facultativement substitué une ou deux fois par un atome d'halogène,
un groupe -C(O)-(CH₂)ₙ-hétéroaryle,
un groupe -C(O)-NH-phényle, dans lequel le groupe phényle est facultativement substitué une ou deux fois par un groupe alkyle, ou
un groupe -S(O)₂-phényle, dans lequel le groupe phényle est facultativement substitué une ou deux fois par un atome d'halogène ; et
n vaut 0 ou 1.

4. Composés selon la revendication 1, dans lesquels
R¹, R² et R³ sont un groupe alkyle ;
X est une simple liaison ;
le cycle A est un cycle saturé à 5 à 7 chaînons, de préférence à 5 à 6 chaînons contenant facultativement un hétéroatome indépendamment choisi parmi un atome d'oxygène, un atome d'azote ou un atome de soufre et les atomes de cycle restants étant des atomes de carbone,
dans lequel ledit cycle A peut être facultativement substitué une ou plusieurs fois par un groupe alkyle,
et si ledit cycle A contient un atome d'azote, ledit atome d'azote peut être facultativement substitué une fois par
un groupe -CH₂-phényle,
un groupe -C(O)-cycloalkyle,
un groupe -C(O)-hétérocyclyle,
un groupe -C(O)-(CH₂)ₙ-phényle, dans lequel le groupe phényle est facultativement substitué une ou deux fois par un atome d'halogène,
un groupe -C(O)-(CH₂)ₙ-thiényle,
un groupe -C(O)-NH-phényle, dans lequel le groupe phényle est facultativement substitué une ou deux fois par un groupe alkyle, ou
un groupe -S(O)₂-phényle, dans lequel le groupe phényle est facultativement substitué une ou deux fois par un atome d'halogène ; et
n vaut 0 ou 1.

5. Composés selon la revendication 1, dans lesquels
R¹ est un atome d'hydrogène ou un groupe alkyle,
dans lequel ledit groupe alkyle est facultativement substitué une fois par -Y-R⁴ ;
Y est une simple liaison ;
R⁴ est un groupe hétérocyclyle ;
R² est un groupe alkyle ;
R³ est un groupe alkyle ;
X est une simple liaison ; et
le cycle A est un cycle hydrocarboné saturé à 5 à 7 chaînons.

6. Composés selon la revendication 5, choisis dans le groupe consistant en :
la 7,7-diméthyl-2-(1,4,5,6-tétrahydro-cyclopentapyrazol-3-yl)-5,7-dihydro-3*H-*imidazo[4,5-*f*]indol-6-one ;
la 7,7-diméthyl-2-(4,5,6,7-tétrahydro-1H-indazol-3-yl)-5,7-dihydro-3H-imidazo[4,5-*f*]indol-6-one ;
la 5,7,7-triméthyl-2-(1,4,5,6-tétrahydro-cyclopentapyrazol-3-yl)-5,7-dihydro-3*H-*imidazo[4,5-*f*]indol-6-one ;
la 5-éthyl-7,7-diméthyl-2-(1,4,5,6-tétrahydro-cyclopentapyrazol-3-yl)-5,7-dihydro-3*H*-imidazo[4,5-*f*]indol-6-one ;
la 7,7-diméthyl-5-propyl-2-(1,4,5,6-tétrahydro-cyclopentapyrazol-3-yl)-5,7-dihydro-3*H*-imidazo[4,5-*f*]indol-6-one ;
la 5-isopropyl-7,7-diméthyl-2-(1,4,5,6-tétrahydro-cyclopentapyrazol-3-yl)-5,7-dihydro-3*H*-imidazo[4,5-*f*]indol-6-one ; et
la 7,7-diméthyl-5-(3-morpholin-4-yl-propyl)-2-(1,4,5,6-tétrahydro-cyclopentapyrazol-3-yl)-5,7-dihydro-3*H*-imidazo[4,5-*f*]indol-6-one ;
la 5-éthyl-7,7-diméthyl-2-(4,5,6,7-tétrahydro-1*H*-indazol-3-yl)-5,7-dihydro-3*H-*imidazo[4,5-*f*]indol-6-one ;
la 5-isopropyl-7,7-diméthyl-2-(4,5,6,7-tétrahydro-1*H*-indazol-3-yl)-5,7-dihydro-3*H-*imidazo[4,5-*f*]indol-6-one ;
la 7,7-diméthyl-5-(3-morpholin-4-yl-propyl)-2-(4,5,6,7-tétrahydro-1*H*-indazol-3-yl)-5,7-dihydro-3*H*-imidazo[4,5-*f*]indol-6-one ; et
la 5,7,7-triéthyl-2-(4,5,6,7-tétrahydro-1*H*-indazol-3-yl)-5,7-dihydro-3*H*-imidazo[4,5-*f*]indol-6-one.

7. Composés selon la revendication 1, dans lesquels
R¹, R² et R³ sont un groupe alkyle ;
X est une simple liaison ; et
le cycle A est un cycle saturé à 5 à 7 chaînons, contenant un hétéroatome indépendamment choisi parmi un atome d'oxygène, un atome d'azote ou un atome de soufre et les atomes de cycle restants étant des atomes de carbone,
dans lequel ledit cycle A peut être facultativement substitué une ou plusieurs fois par un groupe alkyle,
et si ledit cycle A contient un atome d'azote, ledit atome d'azote peut être facultativement substitué une fois par
un groupe -CH₂-phényle,
un groupe -C(O)-cycloalkyle,
un groupe -C(O)-hétérocyclyle,
un groupe -C(O)-(CH₂)ₙ-phényle, dans lequel le groupe phényle est facultativement substitué une ou deux fois par un atome d'halogène,
un groupe -C(O)-(CH₂)ₙ-thiényle,
un groupe -C(O)-NH-phényle, dans lequel le groupe phényle est facultativement substitué une ou deux fois par un groupe alkyle, ou
un groupe -S(O)₂-phényle, dans lequel le groupe phényle est facultativement substitué une ou deux fois par un atome d'halogène ; et
n vaut 0 ou 1.

8. Composés selon la revendication 7, choisis dans le groupe consistant en :
la 5-éthyl-7,7-diméthyl-2-(1,4,6,7-tétrahydro-pyrano[4,3-c]pyrazol-3-yl)-5,7-dihydro-3*H*-imidazo[4,5-f]indol-6-one ;
la 2-(5-benzyl-4,5,6,7-tétrahydro-1*H-*pyrazolo[4,3-c]pyridin-3-yl)-5-éthyl-7,7-diméthyl-5,7-dihydro-3*H*-imidazo[4,5-*f*]indol-6-one ;
la 5-éthyl-7,7-diméthyl-2-(4,5,6,7-tétrahydro-1*H-*pyrazolo[4,3-*c*]pyridin-3-yl)-5,7-dihydro-3*H*-imidazo[4,5-*f*]indol-6-one ;
la 2-(5-benzyl-1,4,5,6-tétrahydro-pyrrolo[3,4-c]pyrazol-3-yl)-5-éthyl-7,7-diméthyl-5,7-dihydro-3*H*-imidazo[4,5-*f*]indol-6-one ;
la 5-éthyl-7,7-diméthyl-2-(1,4,6,7-tétrahydro-thiopyrano[4,3-*c*]pyrazol-3-yl)-5,7-dihydro-3H-imidazo[4,5-*f*]indol-6-one ;
la 2-(5-cyclopropanecarbonyl-1,4,5,6-tétrahydro-pyrrolo[3,4-*c*]pyrazol-3-yl)-5-éthyl-7,7-diméthyl-5,7-dihydro-3H-imidazo[4,5-*f*]indol-6-one ;
la 5-éthyl-2-{5-[2-(4-fluoro-phényl)-acétyl]-4,5,6,7-tétrahydro-1*H-*pyrazolo[4,3-*c*]pyridin-3-yl}-7,7-diméthyl-5,7-dihydro-3*H*-imidazo[4,5-*f*]indol-6-one ;
la 5-éthyl-2-[5-(4-fluoro-benzènesulfonyl)-4,5,6,7-tétrahydro-1*H*-pyrazolo[4,3-*c*]ppidin-3-yl]-7,7-diméthyl-5,7-dihydro-3*H*-imidazo[4,5-*f*]indol-6-one ;
la 5-éthyl-7,7-diméthyl-2-[5-(morpholine-4-carbonyl)-4,5,6,7-tétrahydro-1*H-*pyrazolo[4,3-*c*]pyridin-3-yl]-5,7-dihydro-3*H*-imidazo[4,5-*f*]indol-6-one ;
la 5-éthyl-2-[5-(4-fluoro-benzoyl)-4,5,6,7-tétrahydro-1*H*-pyrazolo[4,3-*c*]ppidin-3-yl]-7,7-diméthyl-5,7-dihydro-3*H*-imidazo[4,5-*f*]indol-6-one ;
la 2-(4,6-dihydro-1H-thiéno[3,4-c]pyrazol-3-yl)-5-éthyl-7,7-diméthyl-5,7-dihydro-3H-imidazo[4,5-f]indol-6-one ;
la 5-éthyl-7,7-diméthyl-2-[5-(2-thiophén-2-yl-acétyl)-1,4,5,6-tétrahydro-pyrrolo[3,4-*c*]pyrazol-3-yl]-5,7-dihydro-3*H*-imidazo[4,5-*f*]indol-6-one ;
la 2-(4,6-dihydro-1*H*-thiéno[3,4-*c*]pyrazol-3-yl)-5-isopropyl-7,7-diméthyl-5,7-dihydro-3*H*-imidazo[4,5-*f*]indol-6-one ;
le (2,6-diéthyl-phényl)-amide d'acide 3-(5-éthyl-7,7-diméthyl-6-oxo-3,5,6,7-tétrahydro-imidazo[4,5-*f*]indol-2-yl)-4,6-dihydro 1*H-*pyrrolo[3,4-*c*]pyrazole-5-carboxylique ;
la 5-éthyl-2-[5-(4-fluoro-benzènesulfonyl)-1,4,5,6-tétrahydro-pyrrolo[3,4-*c*]pyrazol-3-yl]-7,7-diméthyl-5,7-dihydro-3*H*-imidazo[4,5-*f*]indol-6-one ;
le (2,6-diéthyl-phényl)-amide d'acide 3-(5-isopropyl-7,7-diméthyl-6-oxo-3,5,6,7-tétrahydro-imidazo[4,5-*f*]indol-2-yl)-4,6-dihydro-1*H-*pyrrolo[3,4-*c*]pyrazole-5-carboxylique ; et
la 5-isopropyl-7,7-diméthyl-2-[5-(2-thiophèn-2-yl-acétyl)-1,4,5,6-tétrahydro-pyrrolo[3,4-*c*]pyrazol-3-yl]-5,7-dihydro-3*H* imidazo[4,5-*f*]indol-6-one.

9. Procédé de préparation des composés de formule I selon la revendication 1, comprenant les étapes consistant à
a. faire réagir un composé de formule II, 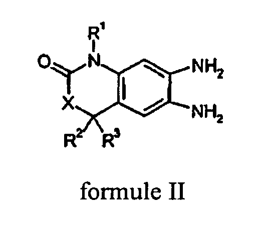 dans laquelle X, R¹, R² et R³ ont la signification donnée ci-dessus dans la revendication 1,
avec un composé de formule III, 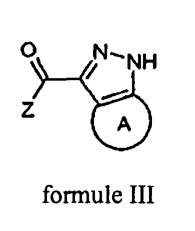 dans laquelle le cycle A a la signification donnée ci-dessus dans la revendication 1 et Z est -OH, -CI, -H, -OMe ou un groupe hydroxybenzotriazole,
pour donner les composés de formule 1 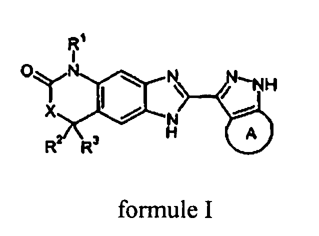
b. isoler les composés de formule I ; et
c. si souhaité, convertir les composés de formule I en leurs sels pharmaceutiquement acceptables.

10. Composition pharmaceutique, contenant un ou plusieurs composés selon les revendications 1 à 8, conjointement avec des excipients pharmaceutiquement acceptables.

11. Composition pharmaceutique, contenant un ou plusieurs composés selon les revendications 1 à 8, pour l'inhibition de la croissance d'une tumeur.

12. Utilisation d'un composé selon les revendications 1 à 8, pour la fabrication de médicaments correspondants pour l'inhibition de la croissance d'une tumeur.
